(19) Europäisches Patentamt
European Patent Office
Office européen des brevets

(11) Veröffentlichungsnummer: **0 233 154 B1**

# (12) EUROPÄISCHE PATENTSCHRIFT

(45) Veröffentlichungstag der Patentschrift: **18.12.91**

(51) Int. Cl.[5]: **C07F 9/38**, C07F 9/40, C07F 9/48, C07F 9/30, C07F 9/32, A61K 31/66

(21) Anmeldenummer: **87810080.9**

(22) Anmeldetag: **10.02.87**

Die Akte enthält technische Angaben, die nach dem Eingang der Anmeldung eingereicht wurden und die nicht in dieser Patentschrift enthalten sind.

## (54) Ungesättigte Aminosäuren.

(30) Priorität: **13.02.86 CH 578/86**

(43) Veröffentlichungstag der Anmeldung:
**19.08.87 Patentblatt 87/34**

(45) Bekanntmachung des Hinweises auf die Patenterteilung:
**18.12.91 Patentblatt 91/51**

(84) Benannte Vertragsstaaten:
**AT BE CH DE ES FR GB GR IT LI LU NL SE**

(56) Entgegenhaltungen:
**GB-A- 2 104 079**

**Chemical Abstracts Band 89, 1978, abstract nr 195 402; & JP-A-7887314, 01.08.1978**

**Chemical Abstracts,band 86, 1977, abstract nr 52479h; B.K. PARK: "Studies on new anti-metabolite produced by microorganism. Part II. 2-Amino-5-phospone-3-pentenoic acid, a new amono acid from N-1409 substance, an antagonist of threonine"; & AGRIC. BIOL.CHEM., 1976, Band 40 (a), Seiten 1905-1906**

(73) Patentinhaber: **CIBA-GEIGY AG**
**Klybeckstrasse 141**
**CH-4002 Basel(CH)**

(72) Erfinder: **Angst, Christof, Dr.**
**13 Lowell Avenue**
**Summit New Jersey(US)**
Erfinder: **Brundish, Derek Edward, Dr.**
**70 Smithbarn**
**Hosham West Sussex RH13 6DU(GB)**
Erfinder: **Dingwall, John Grey, Dr.**
**St. Pantaleonstrasse 16**
**CH-4412 Nuglar(CH)**
Erfinder: **Fagg, Graham Eric, Dr.**
**Wendelinsgasse 1**
**CH-4125 Riehen(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 233 154 B1

## Beschreibung

Die Erfindung betrifft neue ungesättigte Aminosäuren, deren Salze, Verfahren Zur Herstellung dieser neuen Stoffe, pharmazeutische Präparate, die diese Stoffe enthalten und die Verwendung dieser Stoffe und sie enthaltender Präparate.

Bei den erfindungsgemässen Verbindungen handelt es sich um die Verbindungen der Formel I,

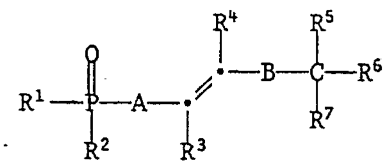

(I)

worin die Kohlenstoff-Kohlenstoff-Doppelbindung trans-konfiguriert ist und $R^1$ Hydroxy oder verethertes Hydroxy bedeutet, $R^2$ Wasserstoff, Alkyl, Hydroxy oder verethertes Hydroxy bedeutet, $R^3$ Wasserstoff, Alkyl, Halogenalkyl, Hydroxyalkyl, Niederalkoxyalkyl, Arylalkyl, Niederalkenyl, Halogen oder Aryl bedeutet, $R^4$ für Wasserstoff, Alkyl oder Aryl steht, $R^5$ Wasserstoff oder Alkyl bedeutet, $R^6$ für Carboxy, verestertes oder amidiertes Carboxy steht, $R^7$ für Amino oder durch Alkyl oder Acyl substituiertes Amino steht, A für unsubstituiertes oder durch Alkyl substituiertes $\alpha,\omega$-Alkylen mit 1 bis 3 Kohlenstoffatomen steht, B eine Bindung bedeutet und "niedere" Reste bis und mit 8 Kohlenstoffatome enthalten, und ihre Salze.

Die Verbindungen der Formel I enthalten mindestens ein chirales Zentrum und können als Enantiomere oder als Enantiomerengemische, wie Racemate, vorliegen, sofern sie mehr als ein chirales Zentrum aufweisen, auch als Diastereomere oder als Diastereomerengemische.

Die Kohlenstoff-Kohlenstoff-Doppelbindung der erfindungsgemässen Verbindungen ist bezüglich $R^3$ und $R^4$ beziehungsweise bezüglich A und B trans-konfiguriert, d.h. es handelt sich bei den Verbindungen der Formel I um die Verbindungen der E-Reihe.

Verbindungen der Formel I, in denen $R^2$ Wasserstoff bedeutet, sind Phosphonige Säuren (engl.: phosphonous acids), solche in denen $R^2$ Alkyl bedeutet, sind Phosphinsäuren (engl.: phosphinic acids), solche in denen $R^2$ für Hydroxy steht, sind Phosphonsäuren (engl.: phosphonic acids). Als Präfixe in der Benennung der Verbindungen der Formel I, die als substituierte Carbonsäuren anzusprechen sind, werden "Phosphino" ($R^2$ bedeutet Wasserstoff), "Phosphonyl" ($R^2$ bedeutet Alkyl) und "Phosphono" ($R^2$ bedeutet Hydroxy) verwendet.

$\alpha,\omega$-Alkylen mit 1 bis 3 Kohlenstoffatomen ist Methylen, 1,2-Ethylen oder 1,3-Propylen. Durch Alkyl substituiertes $\alpha,\omega$-Alkylen ist an einer beliebigen Position substituiert. So bedeutet durch Alkyl substituiertes Methylen z.B. 1,1-Ethylen, 1,1-Butylen oder 1,1-Octylen, durch Alkyl substituiertes 1,2-Ethylen z.B. 1,2-Propylen, 1,2-Butylen, 2,3-Butylen, 1,2-Pentylen oder 1,2-Nonylen, und durch Alkyl substituiertes 1,3-Propylen z.B. 1,3-Butylen, 1,3-Pentylen oder 1,3-Decylen.

Durch Acyl substituiertes Amino $R^7$ kann Acylamino oder Diacylamino sein. Durch Alkyl substituiertes Amino $R^7$ ist Mono- oder Diniederalkylamino.

In einer entsprechenden Acylaminogruppe ist Acyl beispielsweise der Acylrest einer organischen Säure mit z.B. bis zu 18 Kohlenstoffatomen, insbesondere einer gegebenenfalls, z.B. durch Halogen, Amino oder Phenyl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl oder Propionyl, Halogenniederalkanoyl, wie 2-Halogenacetyl, insbesondere 2-Fluor-, 2-Brom-, 2-Iod-, 2,2,2-Trifluor- oder 2,2,2-Trichloracetyl, Aroyl, wie gegebenenfalls substituiertes Benzoyl, z.B. Benzoyl, Halogenbenzoyl, wie 4-Chlorbenzoyl, Niederalkoxybenzoyl, wie 4-Methoxybenzoyl, oder Nitrobenzoyl, wie 4-Nitrobenzoyl. Insbesondere ist auch Niederalkenyloxycarbonyl, z.B. Allyloxycarbonyl, oder gegebenenfalls in 1-oder 2-Stellung substituiertes Niederalkoxycarbonyl geeignet, wie Niederalkoxycarbonyl, z.B. Methoxy- oder Ethoxycarbonyl, gegebenenfalls substituiertes Benzyloxycarbonyl, z.B. Benzyloxycarbonyl oder 4-Nitrobenzyloxycarbonyl, oder Aroylmethoxycarbonyl, worin die Aroylgruppe gegebenenfalls, z.B. durch Halogen, wie Brom substituiertes Benzoyl darstellt, z.B. Phenacyloxycarbonyl oder Bromphenacyloxycarbonyl.

Insbesondere kann in einer entsprechenden Acylaminogruppe Acyl für durch Amino und/oder Phenyl, Carbamoyl, Carboxy, Imidazolyl, Niederalkylthio, Tetrahydropyrrolyl, Hydroxy, Indolyl oder Hydroxyphenyl substituiertes Alkanoylamino stehen, so dass darunter z.B. die Acylreste von Aminosäuren zu verstehen sind, z.B. der natürlich vorkommenden Aminosäuren, wie Alanyl, Asparaginyl, Aspartyl, Glycyl, Histidyl, Isoleucyl, Leucyl, Lysyl, Methionyl, Phenylalanyl, Prolyl, Seryl, Threonyl, Tryptophyl, Tyrosyl oder Valyl;

ebenso fallen darunter die Acylreste von Oligopeptiden, z.B. Di- oder Tripeptiden, wie Oligopeptide aus Alanin, Asparagin oder Asparaginsäure.

In einer Diacylaminogruppe bedeutet Diacyl z.B. zwei Acylreste wie vorstehend definiert, oder Diacyl ist beispielsweise der Acylrest einer organischen Dicarbonsäure mit z.B. bis zu 12 Kohlenstoffatomen, insbesondere einer entsprechenden aromatischen Dicarbonsäure, wie Phthalsäure. Eine solche Gruppe ist in erster Linie Phthalimido.

Verestertes Carboxy ist beispielsweise mit einem aliphatischen oder araliphatischen Alkohol, wie einem gegebenenfalls substituierten Niederalkanol oder Phenylniederalkanol, verestertes Carboxy, wie entsprechendes Niederalkoxy- oder Phenylniederalkoxycarbonyl. Vorzugsweise handelt es sich hierbei um pharmazeutisch annehmbares verestertes Carboxy, wie z.B. verestertes Carboxy, das unter physiologischen Bedingungen in Carboxy übergeführt werden kann. Diese Ester der Formel I können auch als Prodrug-Ester bezeichnet werden.

In pharmazeutisch annehmbarer Weise verestertes Carboxy bedeutet vorzugsweise z.B. Niederalkoxycarbonyl; in höherer als der α-Stellung durch Amino, Mono- oder Diniederalkylamino oder Hydroxy substituiertes Niederalkoxycarbonyl; durch Carboxy substituiertes Niederalkoxycarbonyl, z.B. α-Carboxy-substituiertes Niederalkoxycarbonyl; durch Niederalkoxycarbonyl substituiertes Niederalkoxycarbonyl, z.B. α-Niederalkoxycarbonyl-substituiertes Niederalkoxycarbonyl, Arylniederalkoxycarbonyl, z.B. unsubstituiertes oder substituiertes Benzyloxycarbonyl, oder Pydridylmethoxycarbonyl; Niederalkanoyloxy-substituiertes Methoxycarbonyl, z.B. Pivaloyloxymethoxycarbonyl; durch Niederalkanoyloxy oder Niederalkoxy substituiertes Niederalkoxymethoxycarbonyl; Bicyclo[2.2.1]-heptyloxycarbonyl-substituiertes Methoxycarbonyl, wie Bornyloxycarbonylmethoxycarbonyl; 3-Phthalidoxycarbonyl; durch Niederalkyl, Niederalkoxy oder Halogen substituiertes 3-Phthalidoxycarbonyl; oder Niederalkoxycarbonyloxy-niederalkoxycarbonyl, z.B. 1-(Methoxy- oder Ethoxycarbonyloxy-)ethoxycarbonyl.

Besonders bevorzugte Prodrug-Ester sind z.B. die Niederalkylester mit bis zu vier Kohlenstoffatomen, wie z.B. Butyl- oder Ethylester, die Niederalkanoyloxymethylester, wie z.B. Pivaloyloxymethylester, die in höherer als der α-Stellung durch Diniederalkylamino substituierten Niederalkylester mit zwei bis vier Kohlenstoffatomen in jeder Niederalkylgruppe wie z.B. 2-Diethylaminoethylester, sowie Pyridylmethylester wie 3-Pyridylmethylester.

In amidiertem Carboxy bedeutet die Aminogruppe beispielsweise gegebenenfalls durch Hydroxy monosubstituiertes oder durch aliphatische Reste mono- oder disubstituiertes Amino, wie Amino, Hydroxyamino, Mono- oder Diniederalkylamino oder Niederalkylenamino mit 5 bis 7 Ringgliedern. Vorzugsweise handelt es sich hierbei um pharmazeutisch annehmbares amidiertes Carboxy, wie z.B. um amidiertes Carboxy, das unter physiologischen Bedingungen in Carboxy übergeführt werden kann.

Bevorzugte pharmazeutisch annehmbare Amide sind Verbindungen der Formel I, in denen die Carboxygruppe als Carbamoyl, Niederalkylcarbamoyl, z.B. Ethylcarbamoyl, Diniederalkylcarbamoyl, z.B. Diethylcarbamoyl oder als Diniederalkylamino-niederalkylcarbamoyl, z.B. als (2-Diethylaminoethyl)carbamoyl oder als (3-Diethylaminopropyl)carbamoyl, vorliegt.

Verethertes Hydroxy ist beispielsweise mit einem aliphatischen Alkohol verethertes Hydroxy, wie mit einem gegebenenfalls durch Halogen oder in höherer als der α-Stellung durch Hydroxy, Oxo, Niederalkoxy, Niederalkanoyloxy und/oder Mono- oder Diniederalkylamino substituierten Niederalkanol, Niederalkenol oder Niederalkinol verethertes Hydroxy und bedeutet beispielsweise Niederalkoxy, Halogenniederalkoxy, oder entsprechendes Hydroxy-, Oxo-, Niederalkoxy-, Niederalkanoyloxy oder Mono- oder Diniederalkylaminoniederalkoxy. Verbindungen, in denen $R^1$ und/oder. $R^2$ verethertes Hydroxy bedeuten, sind Ester der phosphorhaltigen Säuregruppe und je nach der Bedeutung von $R^2$ Phosphonigsäureester, Phosphinsäureester oder Phosphonsäureester. Bevorzugte Ester sind die jeweiligen Niederalkylester und Hydroxyniederalkylester.

Salze von erfindungsgemässen Verbindungen sind in erster Linie pharmazeutisch verwendbare, nichttoxische Salze von Verbindungen der Formel I. Solche Salze werden beispielsweise von der in Verbindungen der Formel I vorhandenen Carboxygruppe gebildet und sind in erster Linie Metall- oder Ammoniumsalze, wie Alkalimetall- und Erdalkalimetall-, z.B. Natrium-, Kalium-, Magnesium- oder Calziumsalze, sowie Ammoniumsalze mit Ammoniak oder geeigneten organischen Aminen, wie Niederalkylaminen, z.B. Methylamin, Diethylamin oder Triethylamin, Hydroxyniederalkylaminen, z.B. 2-Hydroxyethylamin, Bis-(2-hydroxyethyl)-amin, Tris-(hydroxymethyl)methylamin oder Tris-(2-hydroxyethyl)-amin, basischen aliphatischen Estern von Carbonsäuren, z.B. 4-Aminobenzoesäure-2-diethylaminoethylester, Niederalkylenaminen, z.B. 1-Ethylpiperidin, Niederalkylendiaminen, z.B. Ethylendiamin, Cycloalkylaminen, z.B. Dicyclohexylamin, oder Benzylaminen, z.B. N,N'-Dibenzylethylendiamin, Benzyltrimethylammoniumhydroxid, Dibenzylamin oder N-Benzyl-β-phenylethylamin. Verbindungen der Formel I mit primärer oder sekundärer Aminogruppe können auch Säureadditionssalze, z.B. mit vorzugsweise pharmazeutisch annehmbaren anorganischen Säuren, wie Halogenwasserstoffsäuren, z.B. Salzsäure oder Bromwasserstoffsäure, Schwefelsäure, Salpetersäure oder

Phosphorsäure, oder mit geeigneten organischen Carbon- oder Sulfonsäuren, z.B. Essigsäure, Propionsäure, Bernsteinsäure, Glykolsäure, Milchsäure, Fumarsäure, Maleinsäure, Weinsäure, Oxalsäure, Zitronensäure, Brenztraubensäure, Benzoesäure, Mandelsäure, Aepfelsäure, Ascorbinsäure, Pamoasäure, Nicotinsäure, Methansulfonsäure, Ethansulfonsäure, Hydroxyethansulfonsäure, Benzolsulfonsäure, 4-Toluolsulfonsäure oder Naphthalinsulfonsäure bilden.

Zur Isolierung oder Reinigung können auch pharmazeutisch ungeeignete Salze Verwendung finden. Zur therapeutischen Anwendung gelangen nur die pharmazeutisch verwendbaren, nicht-toxischen Salze, die deshalb bevorzugt sind.

Aryl steht, auch in Definitionen wie Aroyl oder Arylniederalkoxycarbonyl, für aromatische Kohlenwasserstoffreste, die unsubstituiert oder durch Niederalkyl, Hydroxy, geschütztes Hydroxy, Niederalkoxy, Halogen, Amino, Halogenniederalkyl, Hydroxyniederalkyl, Aminoniederalkyl oder Nitro substituiert sind und bedeutet z.B. unsubstituiertes oder entsprechend substituiertes 1- oder 2-Naphthyl, vorzugsweise jedoch unsubstituiertes oder entsprechend substituiertes Phenyl, wie Phenyl, Niederalkylphenyl, z.B. Methylphenyl, Hydroxyphenyl, Halogenphenyl, z.B. 4-Halogenphenyl, wie 4-Chlorphenyl, Benzyloxyphenyl, Niederalkoxyphenyl, z.B. Methoxyphenyl, Hydroxymethylphenyl, Aminomethylphenyl oder Nitrophenyl.

Die vor- und nachstehend verwendeten Allgemeinbegriffe haben, sofern sie nicht abweichend definiert sind, die folgenden Bedeutungen:

Der Ausdruck "nieder" bedeutet, dass entsprechend definierte Gruppen oder Verbindungen bis und mit 8, vorzugsweise bis und mit 4 Kohlenstoffatome enthalten.

Alkyl bedeutet z.B. Niederalkyl wie Methyl, Ethyl, n-Propyl, Isopropyl, n-Butyl, Isobutyl oder tert.-Butyl, ferner n-Pentyl, n-Hexyl, n-Heptyl oder n-Octyl, bevorzugt Methyl, kann aber auch für $C_9$-$C_{12}$-Alkyl, wie Nonyl, Decyl, Undecyl oder Dodecyl stehen.

Arylalkyl bedeutet z.B. Arylniederalkyl, worin Aryl die vorstehend angegebenen Bedeutungen hat, und steht in erster Linie z.B. für unsubstitutiertes Phenylniederalkyl, wie Benzyl oder 1- oder 2-Phenylethyl.

Niederalkenyl enthält vorzugsweise bis zu 6 Kohlenstoffatome und ist über ein $sp^3$-hybridisiertes Kohlenstoffatom gebunden, wie 2-Propenyl, 2- oder 3-Butenyl oder 3-Pentenyl, kann aber auch Vinyl sein.

Niederalkoxy steht in erster Linie für Methoxy, Ethoxy, n-Propoxy, Isopropoxy, n-Butoxy, Isobutoxy oder tert.-Butoxy.

Halogen hat vorzugsweise eine Atomnummer bis 35 und ist insbesondere Chlor, ferner Fluor oder Brom, kann jedoch auch Iod sein.

Geschütztes Hydroxy ist verestertes, z.B. als Acylgruppe verestertes Hydroxy, wie Niederalkanoyloxy, Benzyloxycarbonyloxy oder Niederalkoxycarbonyloxy, oder verethertes Hydroxy, z.B. 2-Tetrahydropyranyloxy oder Benzyloxy, ferner auch Niederalkoxy.

Halogenniederalkyl ist z.B. Halogenmethyl, wie Fluormethyl, Trifluormethyl oder 1- oder 2-Chlorethyl.

Hydroxyniederalkyl ist z.B. Mono- oder Dihydroxyniederalkyl, trägt die Hydroxygruppe(n) beispielsweise insbesondere in höherer als der α-Stellung, und bedeutet z.B. Hydroxymethyl, 2-Hydroxyethyl, 3-Hydroxy- oder 2,3-Dihydroxy-propyl, 4-Hydroxy- oder 2,4-Dihydroxybutyl, 5-Hydroxy-, 2,5-Dihydroxy- oder 3,5-Dihydroxy-pentyl.

Niederalkoxyniederalkyl ist z.B. Mono- oder Diniederalkoxyniederalkyl, trägt die Niederalkoxygruppe(n) beispielsweise insbesondere in höherer als der α-Stellung und ist beispielsweise 2-Methoxy-, 2-Ethoxy-, 2-Propoxy- oder 2-Isopropoxy-ethyl, 3-Methoxy- oder 3-Ethoxy-propyl oder 3,3-Dimethoxy-, 3,3-Diethoxy-, 2,3-Dimethoxy-oder 2,3-Diethoxypropyl oder 4,4-Dimethoxy-butyl, ferner Methoxy-, Ethoxy-, Dimethoxy- oder Propoxy-oder Isopropoxymethyl.

Aminoniederalkyl ist z.B. Aminomethyl oder 1- oder 2-Aminoethyl.

Niederalkanoyloxy ist z.B. Acetoxy, Propionyloxy, Butyryloxy, ferner Formyloxy oder Pivaloyloxy.

Niederalkoxycarbonyl ist z.B. Methoxycarbonyl oder Ethoxycarbonyl.

Arylniederalkoxycarbonyl ist vorzugsweise Phenylniederalkoxycarbonyl, z.B. Benzyloxycarbonyl oder 1- oder 2-Phenylethyloxycarbonyl.

Mono- oder Diniederalkylamino ist z.B. Methylamino, Dimethylamino, Ethylamino, Diethylamino, Propylamino, Isopropylamino oder Butylamino.

Aus Agric. Biol. Chem. 40, 1905-6(1976), Agric. Biol. Chem. 41, 573-9 (1977) und JP-A1-78/87,314 (Vergl. Chem. Abstr. 89: 195402a (1978)) waren bereits die Z-(D)-2-Amino-5-phosphono-3-pentensäure und ihr Carboxamid als Peptidbaustein der antibiotischen Tripeptide Plumbernycin A bzw. B bekannt. Ferner war, z.B. aus US-PS-4,483,853 und GB-A-2,104,079 bekannt, das aliphatische 2-Amino-ω-phosphonocarbonsäuren N-Methyl-D-asparginsäure (NMDA) an NMDA-sensitiven excitatorischen Aminosäurerezeptoren antagonisieren. Spezifisch war aus GB-A-2,104,079 ferner die 2-Amino-7-phosphono-4-heptensäure vorbekannt.

Die Erfindung beruht auf der Feststellung, dass die Verbindungen der vorliegenden Erfindung aktive

und selektive Antagonisten von N-Methyl-D-aspararaginsäure (NMDA) an NMDA-sensitiven excitatorischen Aminosäure-Rezeptoren von Säugern sind. Sie sind daher geeignet für die Behandlung von Krankheiten, die auf eine Blockierung von NMDA sensitiven Rezeptoren ansprechen, wie z.B. cerebrale Ischämie, Muskelspasmen (Spastizität), Konvulsionen (Epilepsie), Angstzustände oder manische Zustände.

Diese vorteilhaften Wirkungen lassen sich in in vitro- oder in in vivo-Testanordnungen demonstrieren. Dabei werden vorzugsweise Säuger verwendet, z.B. Mäuse, Ratten oder Affen, oder Gewebe oder Enzympräparate von solchen Säugern. Die Verbindungen können enteral oder parenteral verabreicht werden, vorzugsweise oral; oder subkutan, intravenös oder intraperitoneal, z.B. in Gelatinekapseln oder in Form von wässrigen Suspensionen oder Lösungen. Die zu verwendende in vivo-Dosis kann sich zwischen 0,1 und 600 mg/kg bewegen, vorzugsweise zwischen 1 und 300 mg/kg. In vitro können die Verbindungen in Form wässriger Lösungen verwendet werden, wobei sich die Konzentrationen zwischen $10^{-4}$ molaren und $10^{-8}$ molaren Lösungen bewegen können.

Die inhibierende Wirkung auf die NMDA sensitiven excitatorischen Aminosäurerezeptoren lässt sich in vitro bestimmen, indem nach G. Fagg und A. Matus, Proc. Nat. Acad. Sci., USA, 81, 6876-80 (1984), gemessen wird, in welchem Ausmass die Bindung von L-$^3$H-Glutaminsäure an NMDA-sensitiven Rezeptoren inhibiert wird. In vivo lässt sich die inhibierende Wirkung auf NMDA sensitive excitatorische Aminosäurerezeptoren demonstrieren, indem NMDA-induzierte Konvulsionen in der Maus inhibiert werden.

Die antikonvulsiven Eigenschaften der erfindungsgemässen Verbindungen lassen sich ferner durch ihre Wirksamkeit bei der Verhinderung von audiogen hervorgerufenen Anfällen in DBA/2 Mäusen zeigen (Chapman et al., Arzneimittel-Forsch. 34, 1261, 1984).

Die antikonvulsiven Eigenschaften lassen sich ferner duch die Wirksamkeit der erfindungsgemässen Verbindungen als Elektroschockantagonisten an der Maus oder an der Ratte zeigen.

Einen Hinweis auf die anxiolytische Aktivität der Verbindungen der vorliegenden Erfindung gibt ihre gute Wirksamkeit im Konflikt-Modell nach Cook/Davidson (Psychopharmacologia 15, 159-168 (1968)).

Die gute Wirksamkeit der Verbindungen der Formel I hängt in überraschend hohem Ausmass von der Konfiguration an der Doppelbindung ab. So erweist sich das Racemat der aus Agric. Biol. Chem. 41, 573 - 579 (1979), B. K. Park et al., bekannten D-2-Amino-5-phosphono-3-cis-pentensäure z.B. in seiner Fähigkeit, an den NMDA sensitiven Rezeptor zu binden, dem Racemat der erfindungsgemässen 2-Amino-5-phosphono-3-trans-pentensäure (im Beispielteil werden diese Verbindungen als Verbindungen der "E-Reihe" bezeichnet) als weit unterlegen.

Bevorzugt sind Verbindungen der Formel I, worin $R^3$ für Wasserstoff, Alkyl oder Aryl steht.

Ferner bevorzugt sind die Verbindungen der Formel I, worin $R^1$ für Hydroxy, Niederalkoxy oder Hydroxyniederalkoxy steht, $R^2$ Wasserstoff, Alkyl, Hydroxy, Niederalkoxy oder Hydroxyniederalkoxy bedeutet, $R^3$ Wasserstoff, Niederalkyl, Halogenniederalkyl, Hydroxyniederalkyl, Niederalkoxyniederalkyl, unsubstituiertes oder im Phenylteil substituiertes Phenylniederalkyl, Niederalkenyl, Halogen oder unsubstituiertes oder substituiertes Phenyl bedeutet, $R^4$ Wasserstoff, Niederalkyl oder unsubstituiertes oder substituiertes Phenyl bedeutet, $R^5$ für Wasserstoff oder Niederalkyl steht, $R^6$ Carboxy oder pharmazeutisch annehmbares verestertes oder amidiertes Carboxy bedeutet, $R^7$ für Amino, Mono- oder Diniederalkylamino, Alkanoylamino oder durch Halogen, Amino und/oder Phenyl, Carbamoyl, Carboxy, Imidazolyl, Niederalkylthio, Tetrahydropyrrolyl, Hydroxy, Indolyl oder Hydroxyphenyl substituiertes Alkanoylamino, Benzoylamino oder durch Halogen, Niederalkoxy oder Nitro substituiertes Benzoylamino oder Phthalimino steht, A unsubstituiertes oder durch Niederalkyl substituiertes $\alpha,\omega$-Alkylen mit 1 bis 3 Kohlenstoffatomen bedeutet und B eine Bindung bedeutet, worin die Substituenten von Phenyl ausgewählt sind aus der Gruppe bestehend aus Niederalkyl, Hydroxy, Niederalkoxy, Halogen, Amino, Halogenniederalkyl, Hydroxyniederalkyl, Aminoniederalkyl und Nitro, sowie pharmazeutisch annehmbare Salze davon.

Weiterhin bevorzugt sind Verbindungen der Formel I, worin $R^1$ bis $R^5$ wie vorstehend definiert sind, $R^6$ Carboxy, Alkoxycarbonyl oder durch Amino, Mono- oder Diniederalkylamino, Hydroxy oder Niederalkanoyloxy substituiertes Alkoxycarbonyl bedeutet, $R^7$ für Amino, Mononiederalkylamino, Niederalkanoylamino oder Benzoylamino steht, A und B wie vorstehend definiert sind, sowie pharmazeutisch annehmbare Salze davon.

Ebenfalls bevorzugt sind Verbindungen der Formel I, worin $R^1$, $R^2$, $R^5$ bis $R^7$ sowie A und B wie unmittelbar vorstehend definiert sind und worin $R^3$ und $R^4$ unabhängig voneinander Wasserstoff, Niederalkyl, Phenyl oder durch Niederalkyl, Hydroxy, Niederalkoxy, Halogen, Amino, Halogenniederalkyl, Hydroxyniederalkyl, Aminoniederalkyl oder Nitro substituiertes Phenyl bedeuten, und pharmazeutisch annehmbare Salze davon.

Besonders bevorzugt sind Verbindungen der Formel I, worin $R^1$ Hydroxy oder Niederalkoxy bedeutet, $R^2$ für Wasserstoff, Alkyl, Hydroxy oder Niederalkoxy steht, $R^3$ für Wasserstoff, Niederalkyl, Phenyl, Halogenphenyl oder Phenylniederalkyl steht, $R^4$ und $R^5$ Wasserstoff oder Niederalkyl bedeuten, $R^6$ für

Carboxy, Alkoxycarbonyl oder Hydroxyniederalkoxycarbonyl steht, $R^7$ Amino, Mononiederalkylamino, Niederalkanoylamino oder Benzoylamino bedeutet, A für unsubstituiertes oder durch Niederalkyl substituiertes $\alpha,\omega$-Alkylen mit 1 bis 3 Kohlenstoffatomen steht und B eine Bindung bedeutet, und pharmazeutisch annehmbare Salze davon.

Insbesondere bevorzugt sind Verbindungen der Formel I, worin $R^1$ Hydroxy ist, $R^2$ für Wasserstoff, Alkyl oder Hydroxy steht, $R^3$ Wasserstoff, Niederalkyl oder Halogenphenyl bedeutet, $R^4$ für Wasserstoff oder Halogenphenyl und $R^5$ für Wasserstoff stehen, $R^6$ Carboxy, Niederalkoxycarbonyl oder Hydroxyniederalkoxycarbonyl bedeutet, $R^7$ Amino, Mononiederalkylamino, Niederalkanoylamino oder Benzoylamino bedeutet, A für $\alpha,\omega$-Alkylen mit 1 bis 3 Kohlenstoffatomen steht und B eine Bindung bedeutet, und pharmazeutisch annehmbare Salze davon.

Ganz besonders bevorzugt sind die Verbindungen der Formel I, worin $R^1$ Hydroxy ist, $R^2$ für Wasserstoff, Niederalkyl oder Hydroxy steht, $R^3$ Wasserstoff oder Niederalkyl bedeutet, $R^4$ und $R^5$ für Wasserstoff stehen, $R^6$ für Carboxy oder Niederalkoxycarbonyl steht, $R^7$ Amino oder Mononiederalkylamino bedeutet, A für $\alpha,\omega$-Alkylen mit 1 bis 3 Kohlenstoffatomen steht, B eine Bindung bedeutet, sowie pharmazeutisch annehmbare Salze davon.

Herausragend sind die Verbindungen der Formel I, worin $R^1$ und $R^2$ Hydroxy bedeuten, $R^3$ für Wasserstoff oder Niederalkyl steht, $R^4$ und $R^5$ für Wasserstoff stehen, $R^6$ Carboxy bedeutet, $R^7$ für Amino steht, A Methylen und B eine Bindung bedeutet, sowie deren Carbonsäureniederalkylester und pharmazeutisch annehmbare Salze, insbesondere deren bezüglich des Atoms, das die Aminogruppe trägt, R-Enantiomere.

Die Verbindungen der vorliegenden Erfindung lassen sich auf an sich bekannte Weise herstellen, z.B. indem man

a) eine Verbindung der Formel II,

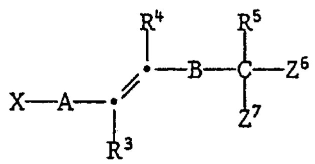

(II)

worin $R^3$, $R^4$, $R^5$ , A und B wie für Formel I definiert sind, $Z^6$ die Bedeutung von $R^6$ hat oder für geschütztes Carboxy steht, $Z^7$ die Bedeutung von $R^7$ hat oder für geschütztes Amino steht und X für reaktionsfähiges verestertes Hydroxy steht, mit einer Verbindung der Formel III,

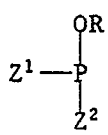

(III)

worin $Z^1$ die Bedeutung von $R^1$ hat oder für geschütztes Hydroxy steht, $Z^2$ die Bedeutung von $R^2$ hat oder für geschützten Wasserstoff oder geschütztes Hydroxy steht und R eine verethernde Gruppe bedeutet, umsetzt, oder

b) um eine Verbindung der Formel I zu erhalten, worin $R^5$ Wasserstoff bedeutet, in einer Verbindung der Formel IV,

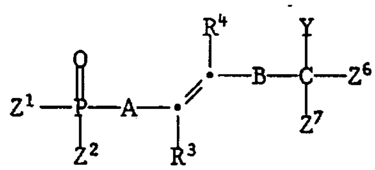

(IV)

worin $R^3$, $R^4$, A und B wie für Formel I definiert sind, $Z^1$ die Bedeutung von $R^1$ hat oder für geschütztes Hydroxy steht, $Z^2$ die Bedeutung von $R^2$ hat oder für geschütztes Hydroxy oder für geschützten Wasserstoff steht, $Z^6$ die Bedeutung von $R^6$ hat oder für geschütztes Carboxy steht, $Z^7$ die Bedeutung von $R^7$ hat oder für geschütztes Amino steht, und Y eine durch Wasserstoff ersetzbare gegebenenfalls veresterte

Carboxygruppe darstellt, die Gruppe Y durch Wasserstoff ersetzt und in einem der vorstehenden Verfahren gegebenenfalls in einer erhaltenen Verbindung geschützte funktionelle Gruppen freisetzt, und gewünschtenfalls eine erhaltene Verbindung der Formel I in eine andere Verbindung der Formel I überführt und/oder gewünschtenfalls eine erhaltene Verbindung der Formel I in ein Salz oder ein erhaltenes Salz in ein anderes Salz oder in eine freie Verbindung der Formel I überführt und/oder gewünschtenfalls ein optisches Isomer aus einer Mischung von stereoisomeren Formen einer erhaltenen Verbindung der Formel I oder eines Salzes davon isoliert.

In den vorstehend definierten Verfahren haben geschütztes Hydroxy und geschütztes Amino z.B. die vorstehend für geschütztes Hydroxy bzw. für durch Acyl substituiertes Amino gegebenen Bedeutungen, insbesondere steht geschütztes Hydroxy hier für Niederalkoxy und ein geschütztes Amino für Niederalkanoylamino. Ein weiterer bevorzugter Vertreter von geschütztem Hydroxy ist z.B. trisubstituiertes Silyl, wie Triniederalkylsilyl, z.B. Trimethylsilyl oder tert.-Butyl-dimethylsilyl.

Darüberhinaus kann geschütztes Amino auch durch substituiertes Niederalkoxycarbonyl substituiertes Amino sein, wie durch 2-Halogenniederalkoxycarbonyl, z.B. 2,2,2-Trichlorethoxycarbonyl, 2-Chlorethoxycarbonyl, 2-Bromethoxycarbonyl oder 2-Iodethoxycarbonyl, oder 2-(trissubstituiertes Silyl)-ethoxycarbonyl, wie 2-Triniederalkylsilylethoxy-carbonyl, z.B. 2-Trimethylsilylethoxycarbonyl oder 2-(Di-n-butyl-methylsilyl)-ethoxycarbonyl, oder 2-Triarylsilylethoxycarbonyl, wie 2-Triphenylsilylethoxycarbonyl substituiertes Amino oder verethertes Mercaptoamino oder Silylamino, oder als Enamino-, Nitro- oder Azidogruppe vorliegen.

Eine veretherte Mercaptoaminogruppe ist in erster Linie eine gegebenenfalls durch Niederalkyl, wie Methyl oder tert.-Butyl, Niederalkoxy, wie Methoxy, Halogen, wie Chlor oder Brom, und/oder Nitro substituierte Phenylthioaminogruppe oder eine Pyridylthioaminogruppe. Entsprechende Gruppen sind beispielsweise 2- oder 4-Nitrophenylthioamino oder 2-Pyridylthioamino.

Eine Silylaminogruppe ist in erster Linie eine organische Silylaminogruppe. In diesen enthält das Siliciumatom vorzugsweise Niederalkyl, z.B. Methyl, Ethyl, n-Butyl oder tert.-Butyl, ferner Aryl, z.B. Phenyl als Substituenten. Geeignete Silylgruppen sind in erster Linie Triniederalkylsilyl, insbesondere Trimethylsilyl oder Dimethyl-tert.-butylsilyl.

Enaminogruppen enthalten an der Doppelbindung in 2-Stellung einen elektronenziehenden Substituenten, beispielsweise eine Carbonylgruppe. Schutzgruppen dieser Art sind beispielsweise 1-Acyl-niederalk-1-en-2-yl-reste, worin Acyl z.B. der entsprechende Rest einer Niederalkancarbonsäure, z.B. Essigsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters, z.B. -methylhalbesters oder -ethylhalbesters, und Niederalk-1-en insbesondere 1-Propen bedeutet. Entsprechende Schutzgruppen sind in erster Linie 1-Niederalkanoyl-prop-1-en-2-yl, z.B. 1-Acetyl-prop-1-en-2-yl, oder 1-Niederalkoxycarbonyl-prop-1-en-2-yl, z.B. 1-Ethoxycarbonyl-prop-1-en-2-yl.

Geschütztes Carboxy ist üblicherweise in veresterter Form geschützt, wobei die Estergruppe unter reduktiven, wie hydrogenolytischen, oder solvolytischen, wie acidolytischen oder hydrolytischen, wie sauer, basisch oder neutral hydrolytischen Bedingungen spaltbar sein kann. Eine geschützte Carboxygruppe kann ferner eine unter physiologischen Bedingungen spaltbare oder eine leicht in eine andere funktionell abgewandelte Carboxygruppe, wie in eine andere veresterte Carboxygruppe, umwandelbare, veresterte Carboxygruppe darstellen.

Solche veresterten Carboxygruppen enthalten als veresternde Gruppen in erster Linie in 1-Stellung verzweigte oder in 1- oder 2-Stellung geeignet substituierte Niederalkylgruppen. Bevorzugte in veresterter Form vorliegende Carboxygruppen sind unter anderen Niederalkoxycarbonyl, z.B. Methoxycarbonyl, Ethoxycarbonyl, 2-Propoxycarbonyl oder tert.-Butoxycarbonyl, und (Hetero-)Arylmethoxycarbonyl mit 1 bis 3 Arylresten oder einem monocyclischen Heteroarylrest, wobei diese gegebenenfalls z.B. durch Niederalkyl, wie tert.-Niederalkyl, z.B. tert.-Butyl, Halogen, z.B. Chlor, und/oder Nitro mono- oder polysubstituiert sind. Beispiele für solche Gruppen sind gegebenenfalls, z.B. wie vorstehend erwähnt, substituiertes Benzyloxycarbonyl, z.B. 4-Nitrobenzyloxycarbonyl, gegebenenfalls, z.B. wie vorstehend erwähnt, substituiertes Diphenylmethoxycarbonyl, z.B. Diphenylmethoxycarbonyl, oder Triphenylmethoxycarbonyl, oder gegebenenfalls, z.B. wie vorstehend erwähnt, substituiertes Picolyloxycarbonyl, z.B. 4-Picolyloxycarbonyl, oder Furfuryloxycarbonyl, wie 2-Furfuryloxycarbonyl.

Geschützter Wasserstoff $Z^1$ ist auf an sich bekannte Weise geschützt, wie beispielsweise in der EP-A-0 009 348 beschrieben. Bei entsprechenden Schutzgruppen handelt es sich vorzugsweise um Gruppen der Formel $-C(C_{1-4}\text{-alkyl})(OR^a)OR^b$, bevorzugt um Gruppen der Formel $-CH(OR^a)OR^b$, worin $R^a$ und $R^b$ jeweils für $C_{1-4}$-Alkyl stehen. Besonders geeignet ist die Gruppe $-CH(OC_2H_5)_2$.

Eine reaktionsfähige veresterte Hydroxygruppe, wie X, ist eine mit einer starken organischen Säure veresterte Hydroxygruppe, z.B. eine mit einer aliphatischen oder aromatischen Sulfonsäure (wie einer

Niederalkansulfonsäure, insbesondere Methansulfonsäure, Trifluormethansulfonsäure, insbesondere Benzolsulfonsäure, p-Toluolsulfonsäure, p-Brombenzolsulfonsäure und p-Nitrobenzolsulfonsäure) oder mit einer starken anorganischen Säure, wie insbesondere Schwefelsäure, oder einer Halogenwasserstoffsäure, wie Chlorwasserstoffsäure oder am meisten bevorzugt Iodwasserstoffsäure oder Bromwasserstoffsäure, veresterte Hydroxygruppe.

Im Verfahren a) bedeutet die verethernde Gruppe R z.B. Phenylniederalkyl, trissubstituiertes Silyl, wie Triniederalkylsilyl oder, vorzugsweise, Alkyl. Die Umsetzung wird auf an sich bekannte Weise durchgeführt, insbesondere unter den bekannten Bedingungen der Michaelis-Arbuzov-Reaktion.

Gemäss einer Variante dieses Verfahrens kann die Umsetzung z.B. eines Trialkylphosphits der Formel III, wie von Triethylphosphit, insbesondere mit Verbindungen der Formel II, in denen A für eine Bindung steht, geeigneterweise katalysiert werden, wie durch ein Halogenid eines Metalls der VIII. Nebengruppe, vorzugsweise einem Nickel-, Palladium- oder Platinhalogenid, insbesondere Nickelchlorid.

In diesem Verfahren können, falls von den Gruppen $Z^1$, $Z^2$, $Z^6$ und $Z^7$ mehrere für geschützte Gruppen stehen, diese vorteilhaft so gewählt werden, dass sie sich in einem einzigen Schritt freisetzen lassen. Bei den in Frage kommenden Bedingungen, unter denen die geschützten Gruppen freigesetzt werden, handelt es sich vorzugsweise um hydrolytische Bedingungen, wie solche einer sauren Hydrolyse, z.B. mit Halogenwasserstoffsäuren, wie mit Salzsäure, vorzugsweise unter Erwärmen.

Die Aufarbeitung erfolgt in an sich bekannter Weise, wobei insbesondere zwei Reinigungsoperationen sich als vorteilhaft erweisen. Entweder kann das Rohprodukt in ein leichtflüchtiges Derivat übergeführt werden, z.B. durch Silylieren, und als solches destillativ gewonnen, danach desilyliert werden. Oder das Rohprodukt kann mit einem Mittel versetzt werden, das mit überschüssiger Säure, wie Halogenwasserstoffsäure, reagiert und sie so entfernt. In Frage kommen z.B. Verbindungen, an die sich eine entsprechende Säure addieren kann, z.B. Niederalkylenoxide (Epoxide), wie Propylenoxid.

Es ist bevorzugt, dieses Verfahren mit Verbindungen der Formeln II und III durchzuführen, in denen $R^3$, $R^4$, $R^5$, A und B wie für Formel I definiert sind, $Z^1$ für geschütztes Hydroxy steht, $Z^2$ für Niederalkyl, geschützten Wasserstoff oder geschütztes Hydroxy steht, R für Niederalkyl steht, $Z^6$ für geschütztes Carboxy steht, $Z^7$ geschütztes Amino und X reaktionsfähiges verestertes Hydroxy bedeuten, und im Anschluss an die Umsetzung, bei der die Verbindung RX frei wird, die geschützten Gruppen freizusetzen. Dabei bedeuten vorzugsweise $Z^1$ Niederalkoxy, $Z^2$ Niederalkyl, Diniederalkoxyniederalkyl oder Niederalkoxy, R Niederalkyl, $Z^6$ Niederalkoxycarbonyl, $Z^7$ Formylamino und X Halogen.

Die Verbindungen der Formel II lassen sich z.B. herstellen, indem N-geschützte Aminomalonsäureester mit Verbindungen der Formel VII

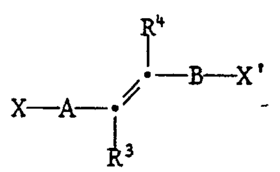

(VII)

auf an sich bekannte Weise umgesetzt werden, worin X und X' unabhängig voneinander reaktionsfähiges verestertes Hydroxy bedeuten. Die so erhaltenen Verbindungen II' lassen sich in Verbindungen der Formel II überführen, indem sie, z.B. wie im Verfahren b) beschrieben, decarboxyliert werden.

Die Ausgangsverbindungen der Formel III sind vorzugsweise Trialkylphosphite ($Z^1$ = Alkoxy, $Z^2$ = Alkoxy, R = Alkyl) oder Alkylphosphonigsäuredialkylester ($Z^1$ = Alkoxy, $Z^2$ = Alkyl, R = Alkyl). Sie sind bekannt oder auf analoge Weise zu bekannten Verfahren herstellbar.

Verbindungen der Formel II, worin A gegebenenfalls durch Alkyl substituiertes Methylen, B eine Bindung, X Halogen und $Z^7$ Formylamino bedeuten, lassen sich z.B. herstellen, indem ein $\alpha,\beta$-ungesättigter Aldehyd, z.B. Acrolein oder Methacrolein, mit einem $\alpha$-Isocyanessigsäurederivat umgesetzt wird, wie z.B. einem $\alpha$-Isocyanessigsäureniederalkylester. Unter geeigneter Katalyse, wie mit niederwertigen Metallsalzen, z.B. Metalloxiden oder Metallhalogeniden, wie Zinkchlorid, Cadmiumchlorid, Silberoxid oder, bevorzugt, Kupferoxid, werden so auf an sich bekannte Weise 5-Vinyl-2-oxazolin-4-carbonsäurederivate, z.B. -ester erhalten, die sich in die offenkettigen Verbindungen der Formel IX überführen lassen,

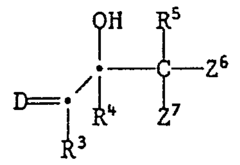

(IX)

worin D für gegebenenfalls durch Alkyl substituiertes Methyliden steht. Diese Verbindungen wiederum können durch selektive Halogenierung, wie Bromierung oder Chlorierung, vorzugsweise unter Kühlen, und unter Verlagerung der Doppelbindung im Sinne einer Allylumlagerung in Verbindungen der Formel II übergeführt werden.

Im Verfahren b) bedeutet die Gruppe Y Carboxy oder verestertes Carboxy wie vorstehend definiert, insbesondere Niederalkoxycarbonyl. Der Ersatz der Gruppe Y durch Wasserstoff kann beispielsweise unter Bedingungen erfolgen, unter denen zuerst verestertes Carboxy verseift und danach Carboxy durch Wasserstoff ersetzt wird (Decarboxylierung), wie unter hydrolytischen Bedingungen, wie solche einer sauren Hydrolyse, z.B. mit Halogenwasserstoffsäuren, wie mit Salzsäure, vorzugsweise unter Erwärmen. In diesem Verfahren können, falls von den Gruppen $Z^1$ , $Z^2$ , $Z^6$ und $Z^7$ mehrere für geschützte Gruppen stehen, diese vorteilhaft so gewählt werden, dass sie sich gemeinsam in dem Schritt freisetzen lassen, bei dem schon die Verseifung und die Decarboxylierung bewirkt wird.

Der Ersatz der Gruppe Y durch Wasserstoff kann auch ohne vorhergehende Verseifung als Dealkoxycarbonylierung erfolgen, beispielsweise nach A. P. Krapcho, Tetrahedron Letters 957 (1973), wie durch Erhitzen in einem wässrigen aprotischen Lösungsmittel, wie Dimethylsulfoxid, in Anwesenheit eines Alkalihalogenids, wie Natriumchlorid.

Es ist bevorzugt, dieses Verfahren mit Verbindungen der Formel IV durchzuführen, in denen $R^3$, $R^4$, A und B wie für Formel I definiert sind, $Z^1$ für geschütztes Hydroxy steht, $Z^2$ für Niederalkyl, geschützten Wasserstoff oder geschütztes Hydroxy steht, $Z^6$ für geschütztes Carboxy steht, $Z^7$ geschütztes Amino und Y eine durch Wasserstoff ersetzbare gegebenenfalls veresterte Carboxygruppe bedeuten, und gemeinsam in dem Schritt, in dem die Gruppe Y durch Wasserstoff ersetzt wird, die geschützten Gruppen freizusetzen. Dabei bedeuten vorzugsweise $Z^1$ Niederalkoxy, $Z^2$ Niederalkyl, Diniederalkoxyniederalkyl oder Niederalkoxy, $Z^6$ und Y Niederalkoxycarbonyl und $Z^7$ Niederalkanoylamino.

Die Verbindungen der Formel IV lassen sich z.B. analog zum Verfahren a) herstellen, indem man eine Verbindung der Formel II'

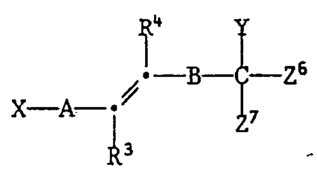

(II')

mit einer Verbindung der Formel III

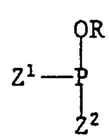

(III)

umsetzt, worin alle Reste die vorstehend angegebenen Bedeutungen aufweisen. Die Verbindungen der Formel II' wiederum lassen sich aus einer Verbindung der Formel VII und einem N-geschützten Aminomalonsäureester herstellen, wie bei Verfahren a) beschrieben.

Zur Ueberführung einer erhaltenen Verbindung der Formel I in eine andere Verbindung der Formel I können Umwandlungen wie die folgenden ausgeführt werden:

Eine Aminogruppe kann alkyliert, und/oder freies Carboxy aus seiner veresterten Form durch Hydrolyse oder Hydrogenolyse freigesetzt, und/oder eine Aminogruppe acyliert und/oder freies Carboxy verestert oder amidiert und/oder an Phosphor gebundenes Hydroxy verestert werden.

Zur Ueberführung einer Aminogruppe in eine Alkylaminogruppe kann die Aminogruppe substitutiv, z.B. mit einem reaktionsfähigen veresterten Alkanol, wie einem Alkylhalogenid, oder reduktiv, wie mit einem

Aldehyd oder Keton, sowie katalytisch aktiviertem Wasserstoff, oder im Fall von Formaldehyd, vorteilhaft mit Ameisensäure als Reduktionsmittel, alkyliert werden.

Freie Carbonsäuren der Formel I oder deren Salze können mit den geeigneten Alkoholen oder entsprechenden Derivaten davon nach bekannten Verfahren in die entsprechenden Ester übergeführt werden, d.h. in Verbindungen der Formel I, die z.B. als Niederalkyl-, Arylniederalkyl-, Niederalkanoyloxymethyl- oder Niederalkoxycarbonylniederalkylester vorliegen.

Zur Veresterung kann eine Carbonsäure direkt mit einem Diazoalkan, insbesondere Diazomethan, oder mit einem entsprechenden Alkohol in Gegenwart eines stark sauren Katalysators (z.B. eine Halogenwasserstoffsäure, Schwefelsäure oder eine organische Sulfonsäure) und/oder eines dehydratisierenden Mittels (z.B. Dicyclohexylcarbodiimid) umgesetzt werden. Alternativ kann die Carbonsäure in ein reaktionsfähiges Derivat übergeführt werden, wie in einen reaktionsfähigen Ester, oder in ein gemischtes Anhydrid, z.B. mit einem Säurehalogenid (z.B. insbesondere ein Säurechlorid), und dieser aktivierte Zwischenstoff wird mit dem gewünschten Alkohol umgesetzt. Veresterungen von an Phosphor gebundenem Hydroxy können wie vorstehend oder auf andere an sich bekannte Weise erfolgen.

Verbindungen der Formel I, worin $R^7$ Amino bedeutet, können in Verbindungen, worin $R^7$ Acylamino bedeutet, übergeführt werden, z.B. unter Verwendung eines entsprechenden Säureanhydrids oder Halogenids, oder vice versa, durch Verfahren, die zum Stand der Technik gehören und hierin im Zusammenhang mit Schutzgruppen beschrieben werden.

Die vorstehenden Reaktionen werden nach Standard-Methoden in Gegenwart oder Abwesenheit von Verdünnungsmitteln, vorzugsweise solchen, die gegenüber den Reagentien inert sind und deren Lösungsmittel darstellen, von Katalysatoren, Kondensationsmitteln bzw. den anderen Mitteln und/oder in inerter Atmosphäre, bei niedrigen Temperaturen, Raumtemperatur oder erhöhten Temperaturen, vorzugsweise beim Siedepunkt der verwendeten Lösungsmittel, bei atmosphärischem oder überatmosphärischem Druck, durchgeführt.

Die Erfindung umfasst weiterhin jegliche Variante der vorliegenden Verfahren, bei der ein bei irgendeiner Stufe derselben erhältliches Zwischenprodukt als Ausgangsmaterial verwendet wird und die verbliebenen Stufen ausgeführt werden, oder das Verfahren bei irgendeiner Stufe desselben unterbrochen wird oder bei der die Ausgangsmaterialien unter Reaktionsbedingungen gebildet werden oder bei der die Reaktionskomponenten in Form ihrer Salze oder optisch reinen Antipoden verwendet werden. Hauptsächlich diejenigen Ausgangsmaterialien sollten bei diesen Reaktionen verwendet werden, die zur Bildung der vorstehend als besonders wertvoll angegebenen Verbindungen führen.

Die Erfindung betrifft auch neue Ausgangsmaterialien und Verfahren zu deren Herstellung.

In Abhängigkeit von der Wahl der Ausgangsmaterialien und Methoden können die neuen Verbindungen in Form eines der möglichen optischen Isomeren oder von deren Gemischen vorliegen, beispielsweise in Abhängigkeit von der Anzahl der asymmetrischen Kohlenstoffatome, als reine optische Isomere, wie als Antipoden, oder als Gemische von optischen Isomeren, wie als Racemate, oder als Gemische von Diastereoisomeren, aus denen ein Antipode, wenn erwünscht, isoliert werden kann.

Erhaltene Gemische von Diastereoisomeren und Gemische der Racemate können wegen der physikochemischen Unterschiede der Bestandteile in bekannter Weise in die reinen Isomere, Diastereoisomere oder Racemate getrennt werden, z.B. durch Chromatographie und/oder fraktionierte Kristallisation.

Die erhaltenen Racemate (racemische Diastereoisomere) können weiterhin in die optischen Antipoden nach an sich bekannten Methoden, beispielsweise durch Umkristallisieren aus einem optisch aktiven Lösungsmittel, mit Hilfe von Mikroorganismen oder durch Umsetzung eines sauren Endprodukts mit einer optisch aktiven Base, die Salze mit der racemischen Säure bildet, und Trennung der auf diese Weise erhaltenen Salze, beispielsweise auf Basis ihrer unterschiedlichen Löslichkeiten, in die Diastereoisomere, aus denen die Antipoden durch Einwirken geeigneter Mittel freigesetzt werden können, aufgetrennt werden. Basische racemische Produkte können gleichfalls in die Antipoden, z.B. durch Trennung von deren diastereoisomeren Salzen, z.B. durch fraktionierte Kristallisation von deren d-oder $\ell$ -Tartraten, getrennt werden. Irgendwelche racemischen Zwischenprodukte oder Ausgangsmaterialien können in ähnlicher Weise getrennt werden.

Schliesslich werden die erfindungsgemässen Verbindungen entweder in freier Form oder in Form von deren Salzen erhalten. Irgendeine erhaltene Base kann in ein entsprechendes Säureadditionssalz, vorzugsweise unter Verwendung einer pharmazeutisch verträglichen Säure oder eines Anionenaustausch-Präparates, übergeführt werden, oder erhaltene Salze können in die entsprechenden freien Basen, beispielsweise unter Verwendung einer stärkeren Base, wie eines Metall- oder Ammoniumhydroxids oder eines basischen Salzes, z.B. eines Alkalimetallhydroxids oder -carbonats, oder eines Kationenaustausch-Präparats, übergeführt werden. Eine Verbindung der Formel I kann auch in die entsprechenden Metall- oder Ammoniumsalze übergeführt werden. Diese oder andere Salze, beispielsweise die Pikrate, können auch für die Reinigung

der erhaltenen Basen verwendet werden. Die Basen werden in die Salze übergeführt, die Salze werden getrennt, und die Basen werden aus den Salzen freigesetzt. Im Hinblick auf die enge Verwandtschaft zwischen den freien Verbindungen und den Verbindungen in Form ihrer Salze ist, wenn immer in dieser Anmeldung eine Verbindung erwähnt wird, auch ein entsprechendes Salz dieser Verbindung mit umfasst, vorausgesetzt, dass dies unter den gegebenen Umständen möglich oder sinnvoll ist.

Die Verbindungen einschliesslich ihrer Salze können auch in Form ihrer Hydrate erhalten werden oder andere für die Kristallisation verwendete Lösungsmittel enthalten.

Die erfindungsgemässen pharmazeutischen Präparate sind solche, die für die enterale, wie die orale oder rektale, und die parenterale Verabreichung an Säuger, einschliesslich des Menschen, zur Behandlung oder Verhinderung von Erkrankungen geeignet sind, die auf die Blockierung von NMDA-Rezeptoren ansprechen, wie z.B. cerebrale Ischämie, Muskelspasmen (Spastizität), Konvulsionen (Epilepsie), Angstzustände oder manische Zustände. Sie umfassen eine wirksame Menge einer pharmakologisch aktiven Verbindung der Formel I oder eines pharmazeutisch verträglichen Salzes derselben, allein oder in Kombination mit einem oder mehreren pharmazeutisch verträglichen Trägern.

Die pharmakologisch aktiven Verbindungen der Erfindung sind bei der Herstellung von pharmazeutischen Zusammensetzungen verwendbar, die eine wirksame Menge derselben allein oder in Verbindung oder Beimischung mit Excipienten oder Trägern, die für die enterale oder parenterale Anwendung geeignet sind, umfassen. Bevorzugt sind Tabletten und Gelatinekapseln, die den aktiven Bestandteil zusammen mit a) Verdünnungsmitteln, z.B. Lactose, Dextrose, Sucrose, Mannit, Sorbit, Cellulose und/oder Glycin, b) Gleitmitteln, z.B. Siliciumdioxid, Talk, Stearinsäure, deren Magnesium- oder Calciumsalz und/oder Polyethylenglykol, für Tabletten auch c) Bindemitteln, z.B. Magnesiumaluminiumsilikat, Stärkepaste, Gelatine, Tragant, Methylcellulose, Natriumcarboxymethylcellulose und/oder Polyvinylpyrrolidon, gewünschtenfalls d) Zerteilungs- bzw. Desintegrationsmitteln, z.B. Stärken, Agar, Alginsäure oder deren Natriumsalz, oder schäumenden Mischungen und/oder e) Absorbentien, farbgebenden Mitteln, Geschmacksstoffen und süssenden Mitteln umfassen. Injizierbare Präparate sind vorzugsweise wässrige isotonische Lösungen oder Suspensionen, und Suppositorien werden vorteilhaft aus Fettemulsionen oder -suspensionen hergestellt. Diese Zusammensetzungen können sterilisiert werden und/oder Adjuvantien, wie Konservierungs-, Stabilisierungs-, Netz- oder Emulgiermittel, Lösungspromotoren, Salze für die Regulierung des osmotischen Drucks und/oder Puffer, enthalten. Zusätzlich können sie auch andere therapeutisch wertvolle Substanzen enthalten. Diese Präparate werden nach herkömmlichen Misch-, Granulier-bzw. Ueberzugsmethoden hergestellt und enthalten etwa 0,1 bis 100 %, vorzugsweise etwa 1 bis 50 %, des aktiven Bestandteils. Eine Einheitsdosis für einen Säuger von etwa 50 bis 70 kg kann zwischen etwa 1 und 500 mg, bevorzugt zwischen etwa 10 und 500 mg, aktiven Bestandteil enthalten.

Die folgenden Beispiele sollen die Erfindung erläutern und keine Einschränkungen darstellen. Die Temperaturen sind in Celsiusgraden angegeben, und sämtliche Teile sind in Form von Gewichtsteilen angegeben. Wenn nicht anders erwähnt, werden sämtliche Verdampfungen unter vermindertem Druck vorzugsweise zwischen etwa 2 und 13 Kilopascal (kPa) durchgeführt.

Beispiel 1:

8,22 g E-2-Formylamino-5-diethylphosphono-3-pentensäureethylester werden in 170 ml 6N Salzsäure gelöst und 22 Stunden unter Rückfluss erhitzt. Nach Einengen im Vakuum wird der ölige Rückstand in wenig Ethanol aufgenommen, und der Ansatz wird wiederum im Vakuum eingedampft. Diese Vorgehensweise wird noch zweimal wiederholt. Der so erhaltene Rückstand wird in 15 ml Ethanol gelöst und tropfenweise mit 20 ml Ethanol/Propylenoxid (1:1) versetzt. Der entstehende bräunlich gefärbte Niederschlag wird abfiltriert und durch Ionentauscher-chromatographie (Dowex® 50W x 8/$H_2O$) gereinigt. Nach Einengen und Lyophilisierung wird die E-2-Amino-5-phosphono-3-pentensäure als weisses amorphes Pulver erhalten, $^1$H-NMR ($D_2O$): 2,39 (dd, 2H, C(5)-H); 4,27(d, 1H, C(2)-H); 5,53 (m, 1H, C(3)-H); 5,87 (m, 1H, C(4)-H), Fp. nach Umkristallisieren aus Ethanol/Wasser 191-192$^\circ$.

Das Ausgangsmaterial wird wie folgt hergestellt:

1,6 g rotes Kupfer(I)oxid werden in 200 ml Benzol vorgelegt. Unter intensivem Rühren wird zu dieser Suspension innerhalb von 10 Minuten eine Lösung von 140 g Isocyanessigsäureethylester und 84 g frisch destilliertem Acrolein in 200 ml Benzol getropft. Die Reaktionstemperatur wird dabei durch Eiskühlung zwischen 30 und 32$^\circ$ gehalten. Nach beendigter Zugabe wird der Ansatz bis zum Abklingen der Exothermie bei 30-32$^\circ$ gehalten, und anschliessend 1 Stunde bei Raumtemperatur gerührt. Nach Abfiltrieren von überschüssigem Kupfer(I)oxid wird das Filtrat im Vakuum bei 30$^\circ$ eingedampft. Der Rückstand wird mit 600 ml Ether versetzt, über Celite filtriert und im Vakuum bis zur Trockne eingedampft. Man erhält so den 5-Vinyl-2oxazolin-4-carbonsäureethylester als blassgelbes Oel, Kp. 100-110$^\circ$ (5,3 Pa).

128 g des 5-Vinyl-2-oxazolin-4-carbonsäureethylester werden in 70 ml Tetrahydrofuran gelöst und mit 27,4 g Wasser und 3,5 g Triethylamin versetzt. Das Reaktionsgemisch wird 62 Stunden bei 65-70° gerührt und nach dem Abkühlen in 200 ml Dichlormethan aufgenommen. Die Lösung wird über 200 g Magnesiumsulfat getrocknet, filtriert und im Vakuum eingedampft. Reinigung des verbliebenen viskosen Oels durch Säulenchromatographie (Silicagel; Hexan/Essigester 3:2) ergibt den 2-Formylamino-3-hydroxy-4-pentensäureethylester als Diastereomerengemisch, Fp. 50-51°.

2,0 g 2-Formylamino-3-hydroxy-4-pentensäureethylester in 80 ml trockenem Tetrahydrofuran werden auf -78° abgekühlt. 2,5 ml Thionylbromid werden langsam so zugetropft, dass die Reaktionstemperatur -75° nicht übersteigt. Nach beendigter Zugabe wird die Reaktionslösung innerhalb von ca. 3 Stunden auf 0° erwärmt und 2,5 Stunden bei dieser Temperatur gerührt. Die orange-gelbe Lösung wird darauf auf 300 ml einer kalten (5-10°) gesättigten wässrigen Natriumhydrogencarbonat-Lösung gegossen und mit Dichlormethan extrahiert. Die organischen Extrakte werden über Magnesiumsulfat getrocknet und bei Raumtemperatur im Vakuum eingedampft. Das verbleibende Oel wird in 20 ml Triethylphosphit gelöst und 2 Stunden im Vakuum (10 kPa) unter Rückfluss (55°) erhitzt. Ueberschüssiges Triethylphosphit wird darauf im Hochvakuum abdestilliert. Durch säulenchromatographische Reinigung (Silicagel, Essigester/Hexan (2:1), dann Essigester) erhält man den E-2-Formylamino-5-diethylphosphono-3-pentensäureethylester als blassgelbes Oel, $^1$H-NMR($CDCl_3$): 2,62 (m, 2H, C(5)-H); 5,19 (m, 1H, C(2)-H), 5,75 (m, 2H, C(3)-H und C(4)-H).

Beispiel 2:

Durch Hydrolyse von E-2-Formylamino-4-methyl-5-diethylphosphono-3-pentensäureethylester auf analoge Weise wie in Beispiel 1 beschrieben wird die E-2-Amino-4-methyl-5-phosphono-3-pentensäure erhalten, $^1$H-NMR ($D_2O$): 1,73 (s, 3H, $CH_3$); 4,55 (s, 1H, C(2)-H).

Das Ausgangsmaterial wird wie folgt hergestellt:

Durch Reaktion von Isocyanessigsäureethylester mit Methacrolein auf analoge Weise wie in Beispiel 1 beschrieben und nach anschliessender fraktionierter Destillation wird der 5-(2-Propenyl)-2-oxazolin-4-carbonsäureethylester als farbloses Oel erhalten, Kp. 110-130° (5,3 Pa).

Durch Hydrolyse des 5-(2-Propenyl)-2-oxazolin-4-carbonsäureethylester auf analoge Weise wie in Beispiel 1 beschrieben wird der 2-Formylamino-3-hydroxy-4-methyl-4-pentensäureethylester erhalten, Fp. 67°.

Durch Reaktion von 2-Formylamino-3-hydroxy-4-methyl-4-pentensäureethylester mit Thionylbromid und anschliessende Behandlung mit Triethylphosphit auf analoge Weise wie in Beispiel 1 beschrieben wird der E-2-Formylamino-4-methyl-5-diethylphosphono-3-pentensäureethylester als blassgelbes Oel erhalten.

Beispiel 3:

Durch Hydrolyse von E-2-Formylamino-5-(O-ethyl-methyl-phosphonyl)-3-pentensäureethylester auf analoge Weise wie in Beispiel 1 beschrieben wird nach Fällung mit Propylenoxid E-2-Amino-5-methylphosphonyl-3-pentensäure als amorphes weisses Pulver erhalten, $^1$H-NMR ($D_2O$): 2,55 (dd, 2H, C(5)-H); 4,38 (d, 1H, C(2)-H); 5,64 (m, 1H, C(3)-H); 5,91 (m, 1H, C(4)-H).

Das Ausgangsmaterial wird wie folgt hergestellt:

Durch Reaktion von E-2-Formylamino-3-hydroxy-4-pentensäureethylester mit Thionylbromid und anschliessende Behandlung mit Methylphosphonigsäurediethylester anstelle von Triethylphosphit auf analoge Weise wie in Beispiel 1 beschrieben wird E-2-Formylamino-5-(O-ethyl-methylphosphonyl)-3-pentensäureethylester als farbloses Oel erhalten, $^1$H-NMR ($CDCl_3$): 2,63 (dd, 2H, C(5)-H); 5,1 (m, 1H, C(2)-H); 5,75 (m, 2H, C(3)-H und C(4)-H).

Beispiel 4:

25 g E-2-Formylamino-5-O-ethyl-diethoxymethylphosphonyl-3-pentensäureethylester werden mit 500 ml 6H Salzsäure 16 Stunden unter Rückfluss gerührt und danach bei 70° im Vakuum eingeengt. Der Rückstand wird in 100 ml 95 % Ethanol/Wasser suspendiert, mit 20 ml Propylenoxid versetzt, das Produkt wird abfiltriert. Nach Umkristallisieren aus Wasser wird E-2-Amino-5-phosphino-3-pentensäure erhalten, Fp. 139-140°.

Das Ausgangsmaterial wird wie folgt hergestellt:

10 g 2-Formylamino-3-hydroxy-4-pentensäureethylester in 50 ml trockenem Tetrahydrofuran werden auf -78° abgekühlt. 12,7 g Thionylchlorid werden so zugetropft, dass die Reaktionstemperatur -75° nicht übersteigt. Danach wird die Reaktionslösung innerhalb 3 Stunden auf -20° erwärmt und 3 Stunden bei

dieser Temperatur gerührt. Die gelbe Lösung wird darauf auf 300 ml einer kalten (5°) gesättigten wässrigen NatriumhydrogencarbonatLösung gegossen und mit Dichlormethan extrahiert. Die organischen Extrakte werden über Natriumsulfat getrocknet und bei 30° im Vakuum eingedampft. Der Rückstand wird durch Säulenchromatographie (Silicagel, Essigester) vorgereinigt, das verbleibende hellgelbe Oel wird in 10 ml Tetrahydrofuran gelöst. Nach Zugabe von 17,0 g Diethoxymethylphosphonigsäure-ethyl-trimethylsilylester wird 24 Stunden bei 35° gerührt. Die dunkelgelbe Lösung wird darauf auf 100 ml einer kalten (5°) gesättigten Natriumhydrogencarbonat-Lösung gegossen und mit Dichlormethan extrahiert. Die organischen Extrakte werden über Natriumsulfat getrocknet und bei 30° im Vakuum eingedampft. Nach Reinigen des Rückstandes durch Säulenchromatographie (Silicagel, Essigester/Methanol) wird der E-2-Formylamino-5-O-ethyl-diethoxymethylphosphonyl-3-pentensäureethylester als hellgelbes Oel erhalten, $^1$H-NMR ($CDCl_3$): 2,70 (m, 2H, C( 5)-H); 4,68 (q, 1H, C(2)-H); 5,20 (m, 1H, (C-P)-H); 5,80 (m, 2H, C(3)-H und C(4)-H).

Beispiel 5:

a) 1,0 g E-2-Amino-5-phosphino-3-pentensäure werden in 20 ml Ethanol suspendiert und mit Chlorwasserstoffgas 2 Stunden bei 65° gesättigt. Nach dem Einengen wird der Rückstand in 10 ml Ethanol gelöst, mit 10 ml Propylenoxid versetzt und der Niederschlag abfiltriert. Nach Umkristallisieren aus Wasser/Aceton 1:1 wird der E-2-Amino-5-phosphino-3-pentensäureethylester erhalten, Fp. 172-173°.

b) 1,0 g E-2-Amino-5-phosphino-3-pentensäure werden in 20 ml n-Butanol suspendiert und mit Chlorwasserstoffgas 3 Stunden bei 60° gesättigt. Nach dem Einengen wird der Rückstand in 15 ml n-Butanol gelöst, mit 10 ml Propylenoxid versetzt und der Niederschlag abfiltriert. Nach Umkristallisieren aus Wasser/Aceton 1:1 wird der E-2-Amino-5-phosphino-3-pentensäurebutylester erhalten, Fp. 160-161°.

Beispiel 6:

a) 2,0 g E-2-Amino-5-phosphono-3-pentensäure werden in 50 ml Ethanol vorgelegt und mit Chlorwasserstoffgas $2\frac{1}{2}$ Stunden bei 50° gesättigt. Nach dem Einengen wird der Rückstand in 18 ml Ethanol gelöst, mit 18 ml Propylenoxid versetzt und der Niederschlag abfiltriert. Nach Umkristallisieren aus Wasser/Ethanol 1:3 wird der 2-Amino-5-phosphono-3-pentensäureethylester erhalten, Fp. 167-168°.

b) 2,0 g E-2-Amino-5-phosphono-3-pentensäure werden in 40 ml n-Butanol suspendiert und mit Chlorwasserstoffgas 3 Stunden bei 40° gesättigt. Nach dem Einengen wird der Rückstand in 30 ml n-Butanol gelöst, mit 15 ml Propylenoxid versetzt und der Niederschlag abfiltriert. Nach Umkristallisieren aus Wasser/Aceton 1:1 wird der E-2-Amino-5-phosphono-3-pentensäurebutylester erhalten, Fp. 160-161°.

c) 2,0 g E-2-Amino-5-phosphono-3-pentensäure werden in 30 ml n-Octanol suspendiert und mit Chlorwasserstoffgas 4 Stunden bei 70° gesättigt. Der Ansatz wird im Vakuum bei 70° auf die Hälfte seines Volumens konzentriert, mit 50 ml Diethylether und 15 ml Propylenoxid versetzt und filtriert. Nach Umkristallisieren aus Wasser/Aceton 1:1 wird der E-2-Amino-5-phosphono-3-pentensäureoctylester erhalten, Fp. 161-162°.

d) 2,0 g 2-Amino-5-phosphono-3-pentensäure werden in 15 ml 1-Dodecanol und 25 ml Tetrahydrofuran suspendiert und mit Chlorwasserstoffgas 4 Stunden bei 50° gesättigt. Der Ansatz wird im Vakuum bei 50° vom Tetrahydrofuran befreit, mit 40 ml Aceton und 20 ml Propylenoxid versetzt und filtriert. Nach Verrühren aus Wasser/Aceton 1:1 wird der E-2-Amino-5-phosphono-3-pentensäuredodecylester erhalten, Fp. 158-159°.

e) 1,5 g E-2-Amino-5-phosphono-3-pentensäure werden in 30 ml n-Propanol suspendiert und mit Chlorwasserstoffgas $2\frac{1}{2}$ Stunden bei 50° gesättigt. Nach dem Einengen wird der Rückstand in 15 ml n-Propanol gelöst, mit 15 ml Propylenoxid versetzt und der Niederschlag abfiltriert. Nach Umkristallisieren aus Wasser/Aceton 1:3 wird der E-2-Amino-5-phosphono-3-pentensäurepropylester erhalten, Fp. 161-162°.

f) 1,5 g 2-Amino-5-phosphono-3-pentensaure werden in 30 ml n-Pentanol suspendiert und mit Chlorwasserstoffgas 3 Stunden bei 50° gesättigt. Nach dem Einengen wird der Rückstand in 15 ml n-Pentanol gelöst, mit 15 ml Propylenoxid versetzt und der Niederschlag abfiltriert. Nach Umkristallisieren aus Wasser/Aceton 1:1 wird der E-2-Amino-5-phosphono-3-pentensäurepentylester erhalten, Fp. 160-161°.

g) 1,5 g E-2-Amino-5-phosphono-3-pentensäure werden in 30 ml iso-Butanol suspendiert und mit Chlorwasserstoffgas $3\frac{1}{2}$ Stunden bei 70° gesättigt. Nach dem Einengen wird der Rückstand in 10 ml iso-Butanol gelöst, mit 10 ml Propylenoxid versetzt und der Niederschlag abfiltriert. Nach Umkristallisieren aus Wasser/Aceton 1:1 wird der E-2-Amino-5-phosphono-3-pentensäureisobutylester erhalten, Fp. 163-164°.

h) 1,5 g E-2-Amino-5-phosphono-3-pentensäure werden in 30 ml sek.-Butanol suspendiert und mit

Chlorwasserstoffgas 4 Stunden bei 75° gesättigt. Nach dem Einengen wird der Rückstand in 10 ml 2-Butanol gelöst, mit 10 ml Propylenoxid versetzt und der Niederschlag abfiltriert. Nach Umkristallisieren aus Wasser/Aceton 1:1 wird der E-2-Amino-5-phosphono-3-pentensäure-sek.-butylester erhalten, Fp. 169-170°.

Beispiel 7:

Herstellung von 1000 Kapseln mit einem Gehalt von jeweils 10 mg der aktiven Substanz des Beispiels 6 mit folgender Zusammensetzung:

| | |
|---|---|
| E-2-Amino-5-phosphono-4-methyl-3-pentensäure | 10,0 g |
| Milchzucker | 207,0 g |
| Modifizierte Stärke | 80,0 g |
| Magnesiumstearat | 3,0 g |

Verfahren:

Sämtliche pulvrigen Bestandteile werden mit einem Sieb von 0,6 mm Maschenweite gesiebt. Dann wird der Wirkstoff in einen geeigneten Mixer gegeben und bis zur Homogenität zuerst mit Magnesiumstearat, dann mit Milchzucker und Stärke vermischt. No. 2 Gelatinekapseln werden mit je 300 mg dieser Mischung gufüllt, wobei man eine Kapselabfüllmaschine verwendet.

Auf analoge Weise werden Kapseln hergestellt, die 10-200 mg der anderen offenbarten und in den Beispielen erwähnten Verbindungen enthalten.

Beispiel 8:

Herstellung von 10'000 Tabletten mit einem Gehalt von je 10 mg der aktiven Susbtanz des Beispiels 6 mit folgender Zusammensetzung:

| | |
|---|---|
| E-2-Amino-5-phosphono-4-methyl-3-pentensäure | 100,00 g |
| Milchzucker | 2,535,00 g |
| Maisstärke | 125,00 g |
| Polyethylenglykol 6000 | 150,00 g |
| Magnesiumstearat | 40,00 g |
| gereinigtes Wasser | q.s. |

Verfahren:

Sämtliche pulvrigen Bestandteile werden mit einem Sieb von 0,6 mm Maschenweite gesiebt. Dann wird der Wirkstoff mit Milchzucker, Magnesiumstearat und mit der Hälfte der Stärke in einem geeigneten Mischer vermischt. Die andere Hälfte der Stärke wird in 65 ml Wasser suspendiert, und die Suspension wird zur siedenden Lösung von Polyethylenglykol in 260 ml Wasser gegeben. Die erhaltene Paste wird zu den Pulvern gegeben und, gegebenenfalls unter Zugabe einer weiteren Wassermenge, granuliert. Das Granulat wird über Nacht bei 35° getrocknet, durch ein Sieb von 1,2 mm Maschenweite getrieben und zu Tabletten gepresst, die eine Bruchrille aufweisen.

Auf analoge Weise werden Tabletten hergestellt, die 10-200 mg einer der anderen offenbarten und in den Beispielen erwähnten Verbindungen enthalten.

Beispiel 9:

Durch Hydrolyse von E-2-Formylamino-4-methyl-5-dimethylphosphono-3-pentensäureethylester auf analoge Weise wie in Beispiel 1 beschrieben wird die E-2-Amino-4-methyl-5-phosphono-3-pentensäure erhalten. $^{1}$H-NMR siehe Beispiel 2. In Vorfraktionen wird die E-2-Amino-4-methyl-5-methylphosphono-3-pentensäure als Nebenprodukt erhalten, Fp. 149-150°.

Das Ausgangsmaterial wird wie folgt hergestellt:
Durch Reaktion von 2-Formylamino-3-hydroxy-4-methyl-4-pentensäureethylester mit Thionylbromid und anschliessende Behandlung mit Trimethylphosphit auf analoge Weise wie in Beispiel 17 beschrieben wird der E-2-Formylamino-4-methyl-5-dimethylphosphono-3-pentensäureethylester als blassgelbes Oel erhalten.

Beispiel 10:

a) 2,0 g E-2-Amino-4-methyl-5-phosphono-3-pentensäure werden in 50 ml Ethanol vorgelegt und mit Chlorwasserstoffgas 2 1/2 Stunden bei 50° gesättigt. Nach dem Einengen wird der Rückstand in 20 ml Ethanol gelöst, mit 20 ml Propylenoxid versetzt und der Niederschlag abfiltriert. Nach Umkristallisieren aus Wasser/Ethanol (1:3) wird der E-2-Amino-4-methyl-5-phosphono-3-pentensäureethylester erhalten, Fp. 193-194°.

Auf analoge Weise werden folgende Ester erhalten:
b) E-2-Amino-4-methyl-5-phosphono-3-pentensäuremethylester, Fp. 193-194°, [Wasser/Aceton (9:1)];
c) E-2-Amino-4-methyl-5-phosphono-3-pentensäure-n-propylester, Fp. 184-185°, (Wasser);
d) E-2-Amino-4-methyl-5-phosphono-3-pentensäure-n-butylester, Fp. 186-187°, [Wasser/Aceton (2:1)];
e) E-2-Amino-4-methyl-5-phosphono-3-pentensäure-iso-butylester, Fp. 181-182°, [Wasser/Aceton (9:1)];
f) E-2-Amino-4-methyl-5-phosphono-3-pentensäure-n-pentylester, Fp. 207-208°;
g) E-2-Amino-4-methyl-5-phosphono-3-pentensäure-n-hexylester, Fp. 207-208°.

Beispiel 11:

21 g E-2-Formylamino-4-methyl-5-O-ethyl-diethoxymethylphosphonyl-3-pentensäureethylester werden mit 400 ml 4,35N Salzsäure 16 Stunden bei 80° gerührt und danach bei 45° im Vakuum eingeengt. Der Rückstand wird in 100 ml Ethanol gelöst und mit 30 ml Propylenoxid versetzt, das Produkt wird abfiltriert. Nach Umkristallisieren aus Wasser wird E-2-Amino-4-methyl-5-phosphino-3-pentensäure erhalten, Fp. 176-177°.

Das Ausgangsmaterial wird wie folgt hergestellt:
50 g 2-Formylamino-3-hydroxy-4-methyl-4-pentensäureethylester in 500 ml trockenem Tetrahydrofuran werden auf -78° abgekühlt. 89 g Thionylchlorid werden so zugetropft, dass die Reaktionstemperatur -70° nicht übersteigt. Danach wird die Reaktionslösung innerhalb 3 Stunden auf -10° erwärmt und 3 Stunden bei dieser Temperatur gerührt, und darauf im Hochvakuum bei 20° eingeengt.

Der Rückstand wird in 400 ml Dichlormethan aufgenommen und mit gesättigter, wässriger Natriumhydrogencarbonatlösung neutralisiert. Die organischen Extrakte werden über Natriumsulfat getrocknet und bei 30° im Vakuum eingedampft. Der Rückstand wird durch Säulenchromatographie (Silicagel, Essigester) vorgereinigt, das verbleibende hellgelbe Oel wird in 30 ml Toluol gelöst. Nach Zugabe von 94 g Diethoxymethylphosphonigsäure-ethyltrimethylsilylester wird 16 Stunden bei 90° gerührt. Die dunkelgelbe Lösung wird auf Eis/Wasser gegossen, mit Natriumhydrogencarbonat neutralisiert und mit Dichlormethan extrahiert. Die organischen Extrakte werden über Natriumsulfat getrocknet und bei 30° im Vakuum eingedampft. Nach Reinigung des Rückstandes durch Säulenchromatographie (Silicagel, Essigester, dann Essigester/Methanol 9:1) wird der E-2-Formylamino-4-methyl-5-0-ethyl-diethoxymethylphosphonyl-3-pentensäure ethylester als hellgelbes Oel erhalten, $^1$H-NMR($CDCl_3$): 2,64 (dd, 2H, C(5)-H); 4,60 (d, 1H, P-CH); 5,26 (m, 2H, C(2)-H und C(3)-H).

Beispiel 12: Racemattrennung der E-2-Amino-4-methyl-5-phosphono-3-pentensäure.

209 mg E-2-Amino-4-methyl-5-phosphono-3-pentensäure in 21 ml 2N Natronlauge werden unter starkem Rühren innerhalb von 20 Minuten bei 20° mit einer Lösung von 1,5 ml Phenylacetylchlorid in 25 ml 1,4-Dioxan versetzt und 4 Stunden bei Raumtemperatur gerührt. Die Reaktionslösung wird auf 250 ml Wasser gegossen und mehrfach mit Dichlormethan extrahiert. Die Wasserphase wird im Vakuum bei 40° auf 20 ml konzentriert, durch Ionenaustauschchromatographie (DOWEX® 50 Wx8/Wasser/1,4-Dioxan 3:1) vorgereinigt und im Vakuum bei 40° eingeengt. Die so erhaltene E-2-Phenylacetylamino-4-methyl-5-phosphono-3-pentensäure wird in 150 ml Wasser mit 2N Natronlauge auf pH 7,5 eingestellt und mit 250 mg EUPERGIT-ACYLASE® 16 Stunden bei 37° gerührt. Nach dem Abfiltrieren im Vakuum bei 40° wird der Ansatz auf 10 ml konzentriert und durch Ionenaustauschchromatographie (DOWEX® 50 Wx8/Wasser) in (D)-E-2-Phenylacetylamino-4-methyl-5-phosphono-3-pentensäure und in (L)-E-2-Amino-4-methyl-5-phosphono-3-pentensäure aufgetrennt.

a) Die Wasserphasen der (L)-E-2-Amino-4-methyl-5-phosphono-3-pentensäure werden im Vakuum eingeengt, und der Rückstand wird durch Umkristallisieren aus Wasser gereinigt, Fp 196°, $[\alpha]_D^{20}$ = +97,1±1,9° (c = 0,5; Wasser).

b) Die Wasserphasen der (D)-E-2-Phenylacetylamino-4-methyl-5-phosphono-3-pentensäure werden im Vakuum eingeengt, der Rückstand wird mit 25 ml 4,35N Salzsäure 3,5 Stunden bei 85° gerührt und darauf mit Dichlormethan mehrfach extrahiert. Nach Einengen der Wasserphasen im Vakuum und Reinigen des Rückstandes durch Ionenaustauschchromatographie wird die (D)-E-2-Amino-4-methyl-5-phosphono-3-pentensäure erhalten, Fp. 194°, $[\alpha]_D^{20}$ = -96,7±1,2° (c = 0,8; Wasser).

Beispiel 13:

2,5 g E-2-Formylamino-5-(O-ethyl-methylphosphonyl)-4-methyl-3-pentensäureethylester werden in 200 ml 4,35 N Salzsäure 26 Stunden unter Stickstoff auf 80° erwärmt. Man dampft im Vakuum ein und löst den Rückstand je zweimal in je 200 ml Wasser, Tetrahydrofuran und Ethanol, wobei die Lösungen jeweils im Vakuum eingedampft werden. Lösen in 150 ml Ethanol, Zugabe von 5 ml Propylenoxid in 100 ml Tetrahydrofuran/ Ethanol (1:1) bei 0° innerhalb von 20 Minuten, Filtrieren des Niederschlages und zwölfstündiges Trocknen bei 50° im Vakuum ergibt die rohe E-2-Amino-4-methyl-5-methylphosphonyl-3-pentensäure, welche durch Chromatographie an 20 g Dowex® 50 Wx8 ($H_2O$) gereinigt wird (amorphes weisses Pulver), $^1$H-NMR($D_2O$): 1,20 (d, 3H, $CH_3$-P); 1,75 (d, 3H, $CH_3$); 2,45 (d, 2H, C(5)-H); 4,50 (d, 1H, C-(2)-H); 5,15 (m, 1H, C(3)-H).

Das Ausgangsmaterial wird durch Reaktion von 2-Formylamino-3-hydroxy-4-methyl-4-pentensäureethylester mit Thionylbromid wie in Beispiel 18 und anschliessende Behandlung mit Methylphosphonigsäurediethylester an Stelle von Triethylphosphit hergestellt.

Beispiel 14:

14,5 g E-2-Formylamino-2-methyl-5-diethylphosphono-3-pentensäuremethylester werden in 500 ml 4,35 N Salzsäure 32 Stunden unter Stickstoff auf 100-105° erwärmt. Aufarbeitung wie in Beispiel 29 ergibt die E-2-Amino-2-methyl-5-phosphono-3-pentensäure, Fp. 225-226° (aus Wasser).

Das Ausgangsmaterial wird wie folgt hergestellt:

Zu einer Lösung vom 17 g wasserfreiem Zinkchlorid in 75 ml Tetrahydrofuran wird bei 0-5° unter Stickstoff innerhalb von 20 Minuten eine Lösung von 14,1 g 2-Isocyanpropionsäuremethylester und 8,5 g frisch destilliertem Acrolein in 50 ml Tetrahydrofuran gegeben, und man lässt 45 Stunden bei 0-5° rühren. Man giesst auf 500 ml 10 % Natriumhydrogencarbonat-Lösung und extrahiert mit 200 ml Dichlormethan. Die organische Phase wird über Natiumsulfat getrocknet und eingedampft. Filtrieren des Rückstandes über Kieselgel (Essigester als Eluens) ergibt den 4-Methyl-5-vinyl-2-oxazolin-4-carbonsäuremethylester. Durch Hydrolyse des 4-Methyl-5-vinyl-2-oxazolin-4-carbonsäuremethylesters auf analoge Weise wie in Beispiel 1 beschrieben wird der 2-Formylamino-2-methyl-3-hydroxy-4-pentensäuremethylester erhalten. Durch Reaktion des 2-Formylamino-3-hydroxy-2-methyl-4-pentensäuremethylesters mit Thionylbromid und anschliessende Behandlung mit Triethylphosphit wie in Beispiel 1 beschrieben wird der E-2-Formylamino-2-methyl-5-diethylphosphono-3-pentensäuremethylester als gelbes Oel erhalten:

Berechnet: C 46,91 % H 7,22 % N 4,56 % P 10,08 %

Gefunden: C 46,1 % H 7,3 % N 4,1 % P 10,6 %

Beispiel 15:

6,3 g E-2-Formylamino-3-methyl-5-diethylphosphono-3-pentensäureethylester werden in 400 ml 4,35 N Salzsäure während 30 Stunden unter Stickstoff auf 100-105° erwärmt. Aufarbeitung wie Beispiel 29 ergibt die E-2-Amino-3-methyl-5-phosphono-3-pentensäure als weisses Pulver, Fp. 168°, $^1$H-NMR ($D_2O$): 1,50 (d, 3H, $CH_3$); 2,4 (m, 2H, $CH_2$); 4,30 (s, 1H, C(2)-H); 5,60 (m, 1H, C(4)-H).

Das Ausgangsmaterial wird wie folgt hergestellt:

Durch Reaktion von Isocyanessigsäureethylester mit Methylvinylketon analog wie in Beispiel 14 beschrieben erhält man den 5-Methyl-5-vinyl-2-oxazolin-4-carbonsäureethylester, Kp. 65-75° (13 Pa). Durch Hydrolyse des 5-Methyl-5-vinyl-2-oxazolin-4-carbonsäureethylesters analog wie in Beispiel 17 beschrieben wird der 2-Formylamino-3-hydroxy-3-methyl-4-pentensäureethylester erhalten. Reaktion des 2-Formylamino-3-hydroxy-3-methyl-4-pentensäureethylesters mit Thionylbromid und anschliessende Behandlung mit Triethylphosphit analog wie in Beispiel 17 beschrieben ergibt den E-2-Formylamino-3-methyl-5-diethylphosphono-3-pentensäureethylester als farblose Flüssigkeit.

Beispiel 16:

E-2-Formylamino-5-diethylphosphono-5-methyl-3-pentensäureethylester wird wie in Beispiel 29 beschrieben mit 4,35 N Salzsäure hydrolysiert. Man isoliert die E-2-Amino-5-methyl-5-phosphono-3-pentensäure als weissen amorphen Festkörper. $^1$H-NMR ($D_2O$): 1,05 (dd, 3H, $CH_3$); 2,45 (m, 1H, C(5)-H); 4,33 (d, 2H, C(2)-H); 5,5 und 5,9 (2m, 2H, C(3)-H und C(4)-H).

Das Ausgangsmaterial wird wie folgt hergestellt:
Reaktion von Crotonaldehyd mit Isocyanessigsäureethylester analog wie in Beispiel 1 beschrieben ergibt den 5-(Propen-1-yl)-2-oxazolin-4-carbonsäureethylester. Durch Hydrolyse des 5-(Propen-1-yl)-2-oxazolin-4-carbonsäureethylesters analog zu Beispiel 1 wird der 2-Formylamino-3-hydroxy-4-hexensäureethylester erhalten. Umsetzung des 2-Formylamino-3-hydroxy-4-hexensäureethylesters mit Thionylbromid und anschliessende Behandlung mit Triethylphosphit analog wie in Beispiel 1 beschrieben (12 Stunden) ergibt den E-2-Formylamino-5-diethylphosphono-5-methyl-3-pentensäureethylester.

Beispiel 17:

Hydrolyse von E-2-Formylamino-4-ethyl-5-dimethylphosphono-3-pentensäureethylester analog wie in Beispiel 29 beschrieben ergibt die E-2-Amino-4-ethyl-5-phosphono-3-pentensäure, Fp. 176° ($H_2O$).

Das Ausgangsmaterial wird wie folgt hergestellt:
Reaktion von 2-Methylen-butyraldehyd mit Isocyanessigsäureethylester analog wie in Beispiel 1 beschrieben ergibt den 5-(Buten-2-yl)-2-oxazolin-4-carbonsäureethylester. Eine Lösung von 16 g 5-(Buten-2-yl)-2-oxazolin-4-carbonsäureethylester in 100 ml Ethanol/Wasser (1:1) wird 15 Stunden unter Rückfluss zum Sieden erwärmt. Eindampfen im Vakuum, Aufnehmen des Rückstandes in 200 ml Dichlormethan, Trocknen über Natriumsulfat, Filtrieren und Eindampfen des Filtrates ergibt den 2-Formylamino-3-hydroxy-4-ethyl-4-pentensäureethylester.

Umsetzung des 2-Formylamino-3-hydroxy-4-ethyl-4-pentensäureethylestersmit Thionylbromid und anschliessende Behandlung mit Trimethylphosphit analog wie in Beispiel 1 beschrieben ergibt den E-2-Formylamino-4-ethyl-5-dimethylphosphono-3-pentensäureethylester.

Beispiel 18:

Hydrolyse von E-2-Formylamino-4-propyl-5-dimethylphosphono-3-pentensäureethylester analog wie in Beispiel 13 beschrieben ergibt die E-2-Amino-4-propyl-5-phosphono-3-pentensäure, Fp. 193° ($H_2O$).

Das Ausgangsmaterial wird wie folgt hergestellt:
Reaktion von 2-Methylen-pentanal mit Isocyanessigsäureethylester analog zu Beispiel 1 ergibt den 5-(Penten-2-yl)-2-oxazolin-4-carbonsäureethylester. Durch Hydrolyse des 5-(Penten-2-yl)-2-oxazolin-4-carbonsäureethylesters analog wie in Beispiel 17 beschrieben wird 2-Formylamino-3-hydroxy-4-propyl-4-pentensäureethylester erhalten. Umsetzung des 2-Formylamino-3-hydroxy-4-propyl-4-pentensäureethylesters mit Thionylbromid und anschliessende Behandlung mit Trimethylphosphit analog wie in Beispiel 1 beschrieben ergibt den E-2-Formylamino-4-propyl-5-dimethylphosphono-3-pentensäureethylester.

Beispiel 19:

Hydrolyse von E-2-Formylamino-4-butyl-5-dimethylphosphono-3-pentensäureethylester analog wie in Beispiel 13 beschrieben ergibt die E-2-Amino-4-butyl-5-phosphono-3-pentensäure, Fp. 186-187° ($H_2O$).

Das Ausgangsmaterial wird wie folgt hergestellt:
Reaktion von 2-Methylen-hexanal mit Isocyanessigsäureethylester analog zu Beispiel 1 ergibt den 5-(Hexen-2-yl)-2-oxazolin-4-carbonsäureethylester, welcher analog wie in Beispiel 17 beschrieben zu 2-Formylamino-3-hydroxy-4-butyl-4-pentensäureethylester hydrolysiert wird. Umsetzung des 2-Formylamino-3-hydroxy-4-butyl-4-pentensäureethylesters mit Thionylbromid und anschliessende Behandlung mit Trimethylphosphit analog zu Beispiel 1 ergibt den E-2-Formylamino-4-butyl-5-dimethylphosphono-3-pentensäureethylester.

Beispiel 20:

Hydrolyse von E-2-Formylamino-4-isopropyl-5-dimethylphosphono-3-pentensäureethylester analog zu Beispiel 13 ergibt die E-2-Amino-4-isopropyl-5-phosphono-3-pentensäure, Fp. 201° ($H_2O$).

Das Ausgangsmaterial wird wie folgt hergestellt:
Reaktion von 3-Methyl-2-methylen-butanal mit Isocyanessigsäureethylester analog zu Beispiel 1 ergibt den

5-( 3-Methyl-buten-2-yl)-2-oxazolin-4-carbonsäureethylester, welcher analog zu Beispiel 17 zu 2-Formylamino-3-hydroxy-4-isopropyl-4-pentensäureethylester hydrolysiert wird. Anschliessende Behandlung mit Thionylbromid gefolgt von Umsetzung mit Trimethylphosphit analog zu Beispiel 1 ergibt den E-2-Formylamino-4-isopropyl-5-dimethylphosphono-3-pentensäureethylester.

Beispiel 21:

3,9 g E-2-Formylamino-4-tert.butyl-5-dimethylphosphono-3-pentensäureethylester werden analog zu Beispiel 29 hydrolysiert. Trennung durch Ionentauscher-Chromatographie (Dowex® W 50, $H_2O$) ergibt 1,8 g E-2-Amino-4-tert.butyl-5-phosphono-3-pentensäure neben 0,075 g Z-2-Amino-4-tert.butyl-5-phosphono-3-pentensäure.
E-Isomer: Fp. 252-253° ($H_2O$); $^1$H-NMR ($D_2O$): 0,95 (s, 9H, $(CH_3)_3C$); 2,65 (m, 2H, $CH_2$); ca. 4,7 (d, 1H, C-(2)-H); 5,33 (m, 1H, C(3)-H)
(Z-Isomer: $^1$H-NMR ($D_2O$): 1,08 (s, 9H, $(CH_3)_3C$); 2,45 (m, 2H, $CH_2$); 4,95 (d, 1H, C(2)-H); 5.20 (m, 1H, C(3)-H)).

Das Ausgangsmaterial wird wie folgt hergestellt:
Reaktion von 3,3-Dimethyl-2-methylen-butanal mit Isocyanessigsäureethylesteranalog wie in Beispiel 1 beschrieben ergibt den 5-(3,3-Dimethyl-buten-2-yl)-2-oxazolin-4-carbonsäureethylester, welcher analog zu Beispiel 17 zu 2-Formylamino-3-hydroxy-4-tert.butyl-4-pentensäureethylester hydrolysiert wird. Anschliessende Umsetzung mit Thionylbromid gefolgt von Behandlung mit Trimethylphosphit analog zu Beispiel 1 ergibt den E-2-Formylamino-4-tert-butyl-5-dimethylphosphono-3-pentensäureethylester.

Beispiel 22:

0,44 g E-2-Formylamino-4-benzyl-5-diethylphosphono-3-pentensäureethylester werden in 8 ml 4,5 N Salzsäure gelöst und 48 Stunden auf 85° erhitzt. Nach Einengen im Vakuum wird der Rückstand in wenig Ethanol gelöst und tropfenweise mit 1 ml Ethanol/Propylenoxid (1:1) versetzt. Der entstehende weisse Niederschlag wird abfiltriert, nach Umkristallisieren aus Wasser wird die E-2-Amino-4-benzyl-5-phosphono-3-pentensäure als farblose Nadeln erhalten, Fp. 196-198°.

Das Ausgangsmaterial wird wie folgt hergestellt:
Durch Reaktion von Isocyanessigsäureethylester mit 2-Benzyl-propenal auf analoge Weise wie in Beispiel 1 beschrieben und nach Reinigung durch Säulenchromatographie (Silicagel; Dichlormethan/Essigester, 98:2) wird der 5-(3-Phenyl-propen-2-yl)-2-oxazolin-4-carbonsäureethylester als farbloses Oel erhalten, $^1$H-NMR ($CDCl_3$): 3,33 (s, 2H, $CH_2$); 4,37 (dd, 1H, C(4)-H); 4,87 (s, 1H), 5,07 (dd, 1H, C(5)-H); 5,16 (s, 1H).

Durch Hydrolyse des 5-( 3-Phenyl-propen-2-yl)-2-oxazolin-4-carbonsäureethylesters auf analoge Weise wie in Beispiel 1 beschrieben wird der 2-Formylamino-3-hydroxy-4-benzyl-4-pentensäureethylester erhalten, Fp. 87-89°.

Durch Reaktion von 2-Formylamino-3-hydroxy-4-benzyl-3-pentensäureethylester mit Thionylbromid und anschliessende Behandlung mit Triethylphosphit bei 100° auf analoge Weise wie in Beispiel 1 beschrieben und nach Chromatographie (Silicagel; Essigester) wird der E-2-Formylamino-4-benzyl-5-diethylphosphono-3-pentensäureethylester als farbloses Oel erhalten, $^1$H-NMR ($CDCl_3$): 2,45 (d, 2H, C(5)-H); 3,80 (s, 1H, $CH_2$); 5,51 (m, 1H, C(3)-H).

Beispiel 23:

0,15 g E-2-Formylamino-4-phenyl-5-diethylphosphono-3-pentensäuremethylester werden in 10 ml 4,5 N Salzsäure gelöst und 192 Stunden auf 75° erhitzt. Nach Einengen im Vakuum wird der schaumige Rückstand in wenig Ethanol gelöst und tropfenweise mit 1 ml Ethanol/Propylenoxid (1:1) versetzt. Der entstehende weisse Niederschlag wird abfiltriert und aus Wasser/Aceton (1:2) umkristallisiert. Man erhält so die E-2-Amino-4-phenyl-5-phosphono-3-pentensäure als farblose Nadeln, Fp. 230-233°.

Das Ausgangsmaterial wird wie folgt hergestellt:
Durch Reaktion von Isocyanessigsäuremethylester mit 2-Phenylacrolein auf analoge Weise wie in Beispiel 1 beschrieben und nach Reinigung durch Säulenchromatographie (Silicagel; Dichlormethan/Methanol 97,5:2,5 wird der 5-(1-Phenyl-vinyl)-2-oxazolin-4-carbonsäuremethylester als blassgelbes Oel erhalten. $^1$H-NMR ($CDCl_3$): 3,80 (s, 3H, $CH_3$); 4,45 (dd, 1H, C(4)-H); 5,76 (d, 1H, C(5)-H).

Durch Hydrolyse des 5-( 1-Phenyl-vinyl)-2-oxazolin-4-carbonsäuremethylesters auf analoge Weise wie in Beispiel 1 beschrieben wird der 2-Formylamino-3-hydroxy-4-phenyl-4-pentensäuremethylester erhalten, Fp. 173-174°.

Durch Reaktion von 2-Formylamino-3-hydroxy-4-phenyl-4-pentensäuremethylester mit Thionylbromid und anschliessende Behandlung mit Triethylphosphit auf analoge Weise wie in Beispiel 1 beschrieben und nach Chromatographie (Silicagel; Essigester/Hexan 4:1) wird der E-2-Formylamino-4-phenyl-5-diethylphosphono-3-pentensäuremethylester als farbloses Oel erhalten, $^1$H-NMR ($CDCl_3$): 2,98 (d, 2H, C(5)-H); 5,03 (dd, 1H, C(2)-H); 5,77 (dd, 1H, C(3)-H).

Beispiel 24:

Zu einer Lösung von 100 mg E-2-Amino-5-phosphono-3-pentensäure in 6 ml Dioxan/Wasser (1:1) gibt man bei 0° 170 mg Natriumhydrogencarbonat und innerhalb von 5 Minuten 50 Mikroliter Acetanhydrid. Man rührt 30 Minuten bei 0°, gibt ca. 2 ml Dowex® 50 $H^+$ zu und filtriert. Das Filtrat wird eingedampft und durch Ionentauscherchromatographie (Dowex® 50 $H^+$) gereinigt. Lyophilisieren der reinen Fraktionen ergibt 110 mg E-2-Acetamino-5-phosphono-3-pentensäure, Fp. 155°.

Soweit dies nicht ausdrücklich ausgeschlossen ist, kann jede der vorstehend beschriebenen Verbindungen der Formel I auch nach jedem anderen der beschriebenen Verfahren hergestellt werden.

**Patentansprüche**

**Patentansprüche für folgende Vertragsstaaten : BE, CH, DE, FR, GB, IT, LI, LU, NL, SE**

1. Verbindungen der Formel I,

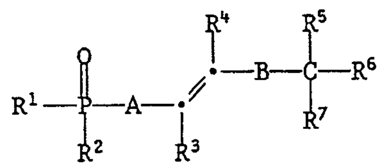

(I)

worin die Kohlenstoff-Kohlenstoff-Doppelbindung trans-konfiguriert ist und $R^1$ Hydroxy oder verethertes Hydroxy bedeutet, $R^2$ Wasserstoff, Alkyl, Hydroxy oder verethertes Hydroxy bedeutet, $R^3$ Wasserstoff, Alkyl, Halogenalkyl, Hydroxyalkyl, Niederalkoxyalkyl, Arylalkyl, Niederalkenyl, Halogen oder Aryl bedeutet, $R^4$ für Wasserstoff, Alkyl oder Aryl steht, $R^5$ Wasserstoff oder Alkyl bedeutet, $R^6$ für Carboxy, verestertes oder amidiertes Carboxy steht, $R^7$ für Amino oder durch Alkyl oder Acyl substituiertes Amino steht, A für unsubstituiertes oder durch Alkyl substituiertes $\alpha,\omega$-Alkylen mit 1 bis 3 Kohlenstoffatomen steht, B eine Bindung bedeutet und "niedere" Reste bis und mit 8 Kohlenstoffatome enthalten, sowie Salze davon.

2. Verbindungen der Formel I gemäss Anspruch 1, worin $R^3$ für Wasserstoff, Alkyl oder Aryl steht.

3. Verbindungen der Formel I gemäss Anspruch 1, worin $R^6$ für Niederalkoxy- oder Phenylniederalkoxycarbonyl steht.

4. Verbindungen der Formel I gemäss Anspruch 1, worin $R^1$ für Hydroxy, Niederalkoxy oder Hydroxyniederalkoxy steht, $R^2$ Wasserstoff, Alkyl, Hydroxy, Niederalkoxy oder Hydroxyniederalkoxy bedeutet, $R^3$ Wasserstoff, Niederalkyl, Halogenniederalkyl, Hydroxyniederalkyl, Niederalkoxyniederalkyl, unsubstituiertes oder im Phenylteil substituiertes Phenylniederalkyl, Niederalkenyl, Halogen oder unsubstituiertes oder substituiertes Phenyl bedeutet, $R^4$ Wasserstoff, Niederalkyl oder unsubstituiertes oder substituiertes Phenyl bedeutet, $R^5$ für Wasserstoff oder Niederalkyl steht, $R^6$ Carboxy oder pharmazeutisch annehmbares verestertes oder amidiertes Carboxy bedeutet, $R^7$ für Amino, Mono- oder Diniederalkylamino, Alkanoylamino oder durch Halogen, Amino und/oder Phenyl, Carbamoyl, Carboxy, Imidazolyl, Niederalkylthio, Tetrahydropyrrolyl, Hydroxy, Indolyl oder Hydroxyphenyl substituiertes Alkanoylamino, Benzoylamino oder durch Halogen, Niederalkoxy oder Nitro substituiertes Benzoylamino oder Phthalimino steht, A unsubstituiertes oder durch Niederalkyl substituiertes $\alpha,\omega$-Alkylen mit 1 bis 3 Kohlenstoffatomen bedeutet und B eine Bindung bedeutet, worin die Substituenten von Phenyl ausgewählt sind aus der Gruppe bestehend aus Niederalkyl, Hydroxy, Niederalkoxy, Halogen, Amino, Halogenniederalkyl, Hydroxyniederalkyl, Aminoniederalkyl und Nitro, sowie pharmazeutisch annehmbare Salze davon.

5. Verbindungen der Formel I gemäss Anspruch 3, worin $R^6$ Carboxy, Alkoxycarbonyl oder durch Amino,

Mono- oder Diniederalkylamino, Hydroxy oder Niederalkanoyloxy substituiertes Alkoxycarbonyl bedeutet, $R^7$ für Amino, Mononiederalkylamino, Niederalkanoylamino oder Benzoylamino steht, sowie pharmazeutisch annehmbare Salze davon.

**6.** Verbindungen der Formel I gemäss Anspruch 4, worin $R^3$ und $R^4$ unabhängig voneinander Wasserstoff, Niederalkyl, Phenyl oder durch Niederalkyl, Hydroxy, Niederalkoxy, Halogen, Amino, Halogenniederalkyl, Hydroxyniederalkyl, Aminoniederalkyl oder Nitro substituiertes Phenyl bedeuten, und pharmazeutisch annehmbare Salze davon.

**7.** Verbindungen der Formel I gemäss Anspruch 1, worin $R^1$ Hydroxy oder Niederalkoxy bedeutet, $R^2$ für Wasserstoff, Alkyl, Hydroxy oder Niederalkoxy steht, $R^3$ für Wasserstoff, Niederalkyl, Phenyl, Halogenphenyl oder Phenylniederalkyl steht, $R^4$ und $R^5$ Wasserstoff oder Niederalkyl bedeuten, $R^6$ für Carboxy, Alkoxycarbonyl oder Hydroxyniederalkoxycarbonyl steht, $R^7$ Amino, Mononiederalkylamino, Niederalkanoylamino oder Benzoylamino bedeutet, A für unsubstituiertes oder durch Niederalkyl substituiertes $\alpha,\omega$-Alkylen mit 1 bis 3 Kohlenstoffatomen steht und B eine Bindung bedeutet, und pharmazeutisch annehmbare Salze davon.

**8.** Verbindungen der Formel I gemäss Anspruch 1, worin $R^1$ Hydroxy ist, $R^2$ für Wasserstoff, Alkyl oder Hydroxy steht, $R^3$ Wasserstoff, Niederalkyl oder Halogenphenyl bedeutet, $R^4$ für Wasserstoff oder Halogenphenyl und $R^5$ für Wasserstoff stehen, $R^6$ Carboxy, Niederalkoxycarbonyl oder Hydroxyniederalkoxycarbonyl bedeutet, $R^7$ Amino, Mononiederalkylamino, Niederalkanoylamino oder Benzoylamino bedeutet, A für $\alpha,\omega$-Alkylen mit 1 bis 3 Kohlenstoffatomen steht und B eine Bindung bedeutet, und pharmazeutisch annehmbare Salze davon.

**9.** Verbindungen der Formel I gemäss Anspruch 1, worin $R^1$ Hydroxy ist, $R^2$ für Wasserstoff, Niederalkyl oder Hydroxy steht, $R^3$ Wasserstoff oder Niederalkyl bedeutet, $R^4$ und $R^5$ für Wasserstoff stehen, $R^6$ für Carboxy oder Niederalkoxycarbonyl steht, $R^7$ Amino oder Mononiederalkylamino bedeutet, A für $\alpha,\omega$-Alkylen mit 1 bis 3 Kohlenstoffatomen steht und B eine Bindung bedeutet, sowie pharmazeutisch annehmbare Salze davon.

**10.** Verbindungen der Formel I gemäss Anspruch 1, worin $R^1$ und $R^2$ Hydroxy bedeuten, $R^3$ für Wasserstoff oder Niederalkyl steht, $R^4$ und $R^5$ für Wasserstoff stehen, $R^6$ Carboxy bedeutet, $R^7$ für Amino steht, A Methylen und B eine Bindung bedeutet, sowie deren Carbonsäureniederalkylester und pharmazeutisch annehmbare Salze.

**11.** E-2-Amino-5-phosphono-3-pentensäure und ihre pharmazeutisch annehmbaren Salze.

**12.** E-2-Amino-4-methyl-5-phosphono-3-pentensäure und ihre pharmazeutisch annehmbaren Salze.

**13.** (D)-E-2-Amino-4-methyl-5-phosphono-3-pentensäure und ihre pharmazeutisch annehmbaren Salze.

**14.** E-2-Amino-4-ethyl-5-phosphono-3-pentensäure und ihre pharmazeutisch annehmbaren Salze.

**15.** E-2-Amino-4-propyl-5-phosphono-3-pentensäure und ihre pharmazeutisch annehmbaren Salze.

**16.** E-2-Amino-4-butyl-5-phosphono-3-pentensäure und ihre pharmazeutisch annehmbaren Salze.

**17.** E-2-Amino-4-isopropyl-5-phosphono-3-pentensäure und ihre pharmazeutisch annehmbaren Salze.

**18.** E-2-Amino-4-benzyl-5-phosphono-3-pentensäure und ihre pharmazeutisch annehmbaren Salze.

**19.** E-2-Amino-4-methyl-5-phosphono-3-pentensäureethylester oder ein Salz davon.

**20.** E-2-Amino-4-methyl-5-phosphono-3-pentensäuremethylester oder ein Salz davon.

**21.** E-2-Amino-4-methyl-5-phosphono-3-pentensäure-n-propylester oder ein Salz davon.

**22.** E-2-Amino-4-methyl-5-phosphono-3-pentensäure-n-butylester oder ein Salz davon.

**23.** E-2-Amino-4-methyl-5-phosphono-3-pentensäure-iso-butylester oder ein Salz davon.

**24.** E-2-Amino-4-methyl-5-phosphono-3-pentensäure-n-pentylester oder ein Salz davon.

**25.** E-2-Amino-4-methyl-5-phosphono-3-pentensäure-n-hexylester oder ein Salz davon.

**26.** Pharmazeutische Präparate enthaltend Verbindungen der Ansprüche 3, 6, 8, 11 und 12 zusammen mit einem pharmazeutisch geeigneten Trägermaterial.

**27.** Pharmazeutische Präparate enthaltend Verbindungen der Ansprüche 1, 4, 5, 7, 9, 10 und 13 bis 25 zusammen mit einem pharmazeutisch geeigneten Trägermaterial.

**28.** Verbindungen gemäss Anspruch 1 zur Anwendung in einem Verfahren zur therapeutischen Behandlung des menschlichen oder tierischen Körpers.

**29.** Verbindungen gemäss Anspruch 1 als Antikonvulsiva.

**30.** Verwendung von Verbindungen gemäss Anspruch 1 von pharmazeutischen Präparaten zur Anwendung als Antikonvulsiva.

**31.** Verfahren zur Herstellung von Verbindungen gemäss Anspruch 1 der Formel I, und deren Salzen, dadurch gekennzeichnet, dass man

a) eine Verbindung der Formel II,

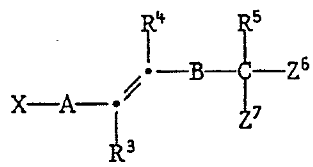

(II)

worin $R^3$, $R^4$, $R^5$, A und B wie für Formel I definiert sind, $Z^6$ die Bedeutung von $R^6$ hat oder für geschütztes Carboxy steht, $Z^7$ die Bedeutung von $R^7$ hat oder für geschütztes Amino steht und X für reaktionsfähiges verestertes Hydroxy steht, mit einer Verbindung der Formel III,

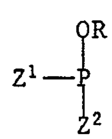

(III)

worin $Z^1$ die Bedeutung von $R^1$ hat oder für geschütztes Hydroxy steht, $Z^2$ die Bedeutung von $R^2$ hat oder für geschützten Wasserstoff oder geschütztes Hydroxy steht und R eine veretherende Gruppe bedeutet, umsetzt, oder

b) um eine Verbindung der Formel I zu erhalten, worin $R^5$ Wasserstoff bedeutet, in einer Verbindung der Formel IV,

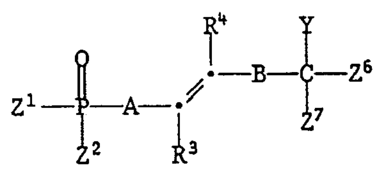

(IV)

worin $R^3$, $R^4$, A und B wie für Formel I definiert sind, $Z^1$ die Bedeutung von $R^1$ hat oder für geschütztes Hydroxy steht, $Z^2$ die Bedeutung von $R^2$ hat oder für geschütztes Hydroxy oder für geschützten Wasserstoff steht, $Z^6$ die Bedeutung von $R^6$ hat oder für geschütztes Carboxy steht, $Z^7$ die Bedeutung von $R^7$ hat oder für geschütztes Amino steht, und Y eine durch Wasserstoff

ersetzbare gegebenenfalls veresterte Carboxygruppe darstellt, die Gruppe Y durch Wasserstoff ersetzt und in einem der vorstehenden Verfahren gegebenenfalls in einer erhaltenen Verbindung geschützte funktionelle Gruppen freisetzt, und gewünschtenfalls eine erhaltene Verbindung der Formel I in eine andere Verbindung der Formel I überführt und/oder gewünschtenfalls eine erhaltene Verbindung der Formel I in ein Salz oder ein erhaltenes Salz in ein anderes Salz oder in eine freie Verbindung der Formel I überführt und/oder gewünschtenfalls ein optisches Isomer aus einer Mischung von stereoisomeren Formen einer erhaltenen Verbindung der Formel I oder eines Salzes davon isoliert.

**32.** Verfahren nach Anspruch 31, dadurch gekennzeichnet, dass man eine Verbindung der Formel II,

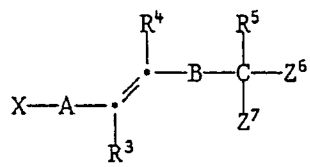

(II)

worin $R^3$, $R^4$, $R^5$, A und B wie für Formel I definiert sind, $Z^6$ für geschütztes Carboxy steht, $Z^7$ für geschütztes Amino steht und X für reaktionsfähiges verestertes Hydroxy steht, mit einer Verbindung der Formel III,

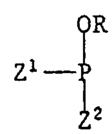

(III)

worin $Z^1$ für geschütztes Hydroxy steht, $Z^2$ für Niederalkyl, geschützten Wasserstoff oder geschütztes Hydroxy steht und R Niederalkyl bedeutet, umsetzt und im Anschluss an diese Umsetzung die geschützten Gruppen freisetzt.

**33.** Verfahren nach Anspruch 32, worin $Z^1$ Niederalkoxy, $Z^2$ Niederalkyl, Diniederalkoxyniederalkyl oder Niederalkoxy, R Niederalkyl, $Z^6$ Niederalkoxycarbonyl, $Z^7$ Formylamino und X Halogen bedeuten.

**34.** Verfahren nach Anspruch 31 zur Umwandlung einer Verbindung der Formel I, worin $R^6$ Carboxy bedeutet, in eine Verbindung der Formel I, worin $R^6$ verestertes Carboxy bedeutet.

**Patentansprüche für folgende Vertragsstaaten : AT, ES, GR**

**1.** Verfahren zur Herstellung von Verbindungen der Formel I,

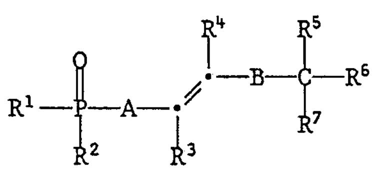

(I)

worin die Kohlenstoff-Kohlenstoff-Doppelbindung trans-konfiguiert ist und $R^1$ Hydroxy oder verethertes Hydroxy bedeutet, $R^2$ Wasserstoff, Alkyl, Hydroxy oder verethertes Hydroxy bedeutet, $R^3$ Wasserstoff, Alkyl, Halogenalkyl, Hydroxyalkyl, Niederalkoxyalkyl, Arylalkyl, Niederalkenyl, Halogen oder Aryl bedeutet, $R^4$ für Wasserstoff, Alkyl oder Aryl steht, $R^5$ Wasserstoff oder Alkyl bedeutet, $R^6$ für Carboxy, verestertes oder amidiertes Carboxy steht, $R^7$ für Amino oder durch Alkyl oder Acyl substituiertes Amino steht, A für unsubstituiertes oder durch Alkyl substituiertes $\alpha,\omega$-Alkylen mit 1 bis 3 Kohlenstoffatomen steht, B eine Bindung bedeutet, und "niedere" Reste bis und mit 8 Kohlenstoffatome enthalten, sowie ihrer Salze, dadurch gekennzeichnet, dass man

a) eine Verbindung der Formel II,

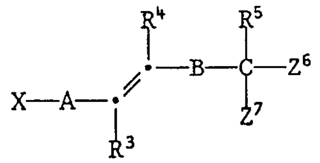

(II)

worin $R^3$, $R^4$, $R^5$, A und B wie für Formel I definiert sind, $Z^6$ die Bedeutung von $R^6$ hat oder für geschütztes Carboxy steht, $Z^7$ die Bedeutung von $R^7$ hat oder für geschütztes Amino steht und x für reaktionsfähiges verestertes Hydroxy steht, mit einer Verbindung der Formel III,

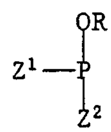

(III)

worin $Z^1$ die Bedeutung von $R^1$ hat oder für geschütztes Hydroxy steht, $Z^2$ die Bedeutung von $R^2$ hat oder für geschützten Wasserstoff oder geschütztes Hydroxy steht und R eine veräthernde Gruppe bedeutet, umsetzt, oder

b) um eine Verbindung der Formel I zu erhalten, worin $R^5$ Wasserstoff bedeutet, in einer Verbindung der Formel IV,

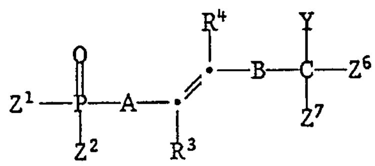

(IV)

worin $R^3$, $R^4$, A und B wie für Formel I definiert sind, $Z^1$ die Bedeutung von $R^1$ hat oder für geschütztes Hydroxy steht, $Z^2$ die Bedeutung von $R^2$ hat oder für geschütztes Hydroxy oder für geschützten Wasserstoff steht, $Z^6$ die Bedeutung von $R^6$ hat oder für geschütztes Carboxy steht, $Z^7$ die Bedeutung von $R^7$ hat oder für geschütztes Amino steht, und Y eine durch Wasserstoff ersetzbare gegebenenfalls veresterte Carboxygruppe darstellt, die Gruppe Y durch Wasserstoff ersetzt, und in einem der vorstehenden Verfahren gegebenenfalls in einer erhaltenen Verbindung geschützte funktionelle Gruppen freisetzt, und gewünschtenfalls eine erhaltene Verbindung der Formel I in eine andere Verbindung der Formel I überführt und/oder gewünschtenfalls eine erhaltene Verbindung der Formel I in ein Salz oder ein erhaltenes Salz in ein anderes Salz oder in eine freie Verbindung der Formel I überführt und/oder gewünschtenfalls ein optisches Isomer aus einer Mischung von stereoisomeren Formen einer erhaltenen Verbindung der Formel I oder eines Salzes davon isoliert.

**2.** Verfahren nach Anspruch 1, dadurch gekennzeichnet, dass man eine Verbindung der Formel II,

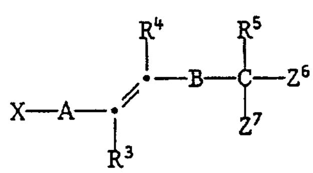

(II)

worin $R^3$, $R^4$ , $R^5$, A und B wie für Formel I definiert sind, $Z^6$ für geschütztes Carboxy steht, $Z^7$ für geschütztes Amino steht und X für reaktionsfähiges verestertes Hydroxy steht, mit einer Verbindung der Formel III,

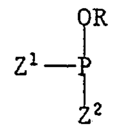 (III)

worin $Z^1$ für geschütztes Hydroxy steht, $Z^2$ für Niederalkyl, geschützten Wasserstoff oder geschütztes Hydroxy steht und R Niederalkyl bedeutet, umsetzt und im Anschluss an diese Umsetzung die geschützten Gruppen freisetzt.

3. Verfahren nach Anspruch 2, worin $Z^1$ Niederalkoxy, $Z^2$ Niederalkyl, Diniederalkoxyniederalkyl oder Niederalkoxy, R Niederalkyl, $Z^6$ Niederalkoxycarbonyl, $Z^7$ Formylamino und X Halogen bedeuten.

4. Verfahren nach Anspruch 1 zur Umwandlung einer Verbindung der Formel I, worin $R^6$ Carboxy bedeutet, in eine Verbindung der Formel I, worin $R^6$ verestertes Carboxy bedeutet.

5. Verfahren gemäss einem der Ansprüche 1 bis 4 zur Herstellung von Verbindungen der Formel I gemäss Anspruch 1, worin $R^3$ für Wasserstoff, Alkyl oder Aryl steht.

6. Verfahren gemäss einem der Ansprüche 1 bis 4 zur Herstellung von Verbindungen der Formel I gemäss Anspruch 1, worin $R^6$ für Niederalkoxy- oder Phenylniederalkoxycarbonyl steht.

7. Verfahren gemäss einem der Ansprüche 1 bis 4 zur Herstellung von Verbindungen der Formel I, worin $R^1$ für Hydroxy, Niederalkoxy oder Hydroxyniederalkoxy steht, $R^2$ Wasserstoff, Alkyl, Hydroxy, Niederalkoxy oder Hydroxyniederalkoxy bedeutet, $R^3$ Wasserstoff, Niederalkyl, Halogenniederalkyl, Hydroxyniederalkyl, Niederalkoxyniederalkyl, unsubstituiertes oder im Phenylteil substituiertes Phenylniederalkyl, Niederalkenyl, Halogen oder unsubstituiertes oder substituiertes Phenyl bedeutet, $R^4$ Wasserstoff, Niederalkyl oder unsubstituiertes oder substituiertes Phenyl bedeutet, $R^5$ für Wasserstoff oder Niederalkyl steht, $R^6$ Carboxy oder pharmazeutisch annehmbares verestertes oder amidiertes Carboxy bedeutet, $R^7$ für Amino, Mono- oder Diniederalkylamino, Alkanoylamino oder durch Halogen, Amino und/oder Phenyl, Carbamoyl, Carboxy, Imidazolyl, Niederalkylthio, Tetrahydropyrrolyl, Hydroxy, Indolyl oder Hydroxyphenyl substituiertes Alkanoylamino, Benzoylamino oder durch Halogen, Niederalkoxy oder Nitro substituiertes Benzoylamino oder Phthalimino steht, A unsubstituiertes oder durch Niederalkyl substituiertes $\alpha,\omega$-Alkylen mit 1 bis 3 Kohlenstoffatomen bedeutet und B eine Bindung bedeutet, worin die Substituenten von Phenyl ausgewählt sind aus der Gruppe bestehend aus Niederalkyl, Hydroxy, Niederalkoxy, Halogen, Amino, Halogenniederalkyl, Hydroxyniederalkyl, Aminoniederalkyl und Nitro, sowie pharmazeutisch annehmbarer Salze davon.

8. Verfahren gemäss Anspruch 7 zur Herstellung von Verbindungen der Formel I, worin $R^6$ Carboxy, Alkoxycarbonyl oder durch Amino, Mono- oder Diniederalkylamino, Hydroxy oder Niederalkanoyloxy substituiertes Alkoxycarbonyl bedeutet, $R^7$ für Amino, Mononiederalkylamino, Niederalkanoylamino oder Benzoylamino steht, sowie pharmazeutisch annehmbarer Salze davon.

9. Verfahren gemäss Anspruch 8 zur Herstellung von Verbindungen der Formel I, worin $R^3$ und $R^4$ unabhängig voneinander Wasserstoff, Niederalkyl, Phenyl oder durch Niederalkyl, Hydroxy, Niederalkoxy, Halogen, Amino, Halogenniederalkyl, Hydroxyniederalkyl, Aminoniederalkyl oder Nitro substituiertes Phenyl bedeuten, und pharmazeutisch annehmbarer Salze davon.

10. Verfahren gemäss einem der Ansprüche 1 bis 4 zur Herstellung von Verbindungen der Formel I, worin $R^1$ Hydroxy oder Niederalkoxy bedeutet, $R^2$ für Wasserstoff, Alkyl, Hydroxy oder Niederalkoxy steht, $R^3$ für Wasserstoff, Niederalkyl, Phenyl, Halogenphenyl oder Phenylniederalkyl steht, $R^4$ und $R^5$ Wasserstoff oder Niederalkyl bedeuten, $R^6$ für Carboxy, Alkoxycarbonyl oder Hydroxyniederalkoxycarbonyl steht, $R^7$ Amino, Mononiederalkylamino, Niederalkanoylamino oder Benzoylamino bedeutet, A für unsubstituiertes oder durch Niederalkyl substituiertes $\alpha,\omega$-Alkylen mit 1 bis 3 Kohlenstoffatomen steht und B eine Bindung bedeutet, und pharmazeutisch annehmbarer Salze davon.

11. Verfahren gemäss einem der Ansprüche 1 bis 4 zur Herstellung von Verbindungen der Formel I, worin $R^1$ Hydroxy ist, $R^2$ für Wasserstoff, Alkyl oder Hydroxy steht, $R^3$ Wasserstoff, Niederalkyl oder

Halogenphenyl bedeutet, $R^4$ für Wasserstoff oder Halogenphenyl und $R^5$ für Wasserstoff stehen, $R^6$ Carboxy, Niederalkoxycarbonyl oder Hydroxyniederalkoxycarbonylbedeutet, $R^7$ Amino, Mononiederalkylamino, Niederalkanoylamino oder Benzoylamino bedeutet, A für $\alpha,\omega$-Alkylen mit 1 bis 3 Kohlenstoffatomen steht und B eine Bindung bedeutet, und pharmazeutisch annehmbarer Salze davon.

**12.** Verfahren gemäss einem der Ansprüche 1 bis 4 zur Herstellung von Verbindungen der Formel I, worin $R^1$ Hydroxy ist, $R^2$ für Wasserstoff, Niederalkyl oder Hydroxy steht, $R^3$ Wasserstoff oder Niederalkyl bedeutet, $R^4$ und $R^5$ für Wasserstoff stehen, $R^6$ für Carboxy oder Niederalkoxycarbonyl steht, $R^7$ Amino oder Mononiederalkylamino bedeutet, A für $\alpha,\omega$-Alkylen mit 1 bis 3 Kohlenstoffatomen steht und B eine Bindung bedeutet, sowie pharmazeutisch annehmbarer Salze davon.

**13.** Verbindungen der Formel I gemäss Anspruch 1, worin $R^1$ und $R^2$ Hydroxy bedeuten, $R^3$ für Wasserstoff oder Niederalkyl steht, $R^4$ und $R^5$ für Wasserstoff stehen, $R^6$ Carboxy bedeutet, $R^7$ für Amino steht, A Methylen und B eine Bindung bedeutet, und ihrer Carbonsäureniederalkylester und pharmazeutisch annehmbaren Salze.

**14.** Verfahren gemäss einem der Ansprüche 1 bis 4 zur Herstellung von E-2-Amino-5-phosphono-3-pentensäure und ihrer pharmazeutisch annehmbaren Salze.

**15.** Verfahren gemäss einem der Ansprüche 1 bis 4 zur Herstellung von E-2-Amino-4-methyl-5-phosphono-3-pentensäure und ihrer pharmazeutisch annehmbaren Salze.

**16.** Verfahren gemäss einem der Ansprüche 1 bis 4 zur Herstellung von (D)-E-2-Amino-4-methyl-5-phosphono-3-pentensäure und ihrer pharmazeutisch annehmbaren Salze.

**17.** Verfahren gemäss einem der Ansprüche 1 bis 4 zur Herstellung von E-2-Amino-4-ethyl-5-phosphono-3-pentensäure und ihrer pharmazeutisch annehmbaren Salze.

**18.** Verfahren gemäss einem der Ansprüche 1 bis 4 zur Herstellung von E-2-Amino-4-propyl-5-phosphono-3-pentensäure und ihrer pharmazeutisch annehmbaren Salze.

**19.** Verfahren gemäss einem der Ansprüche 1 bis 4 zur Herstellung von E-2-Amino-4-butyl-5-phosphono-3-pentensäure und ihrer pharmazeutisch annehmbaren Salze.

**20.** Verfahren gemäss einem der Ansprüche 1 bis 4 zur Herstellung von E-2-Amino-4-isopropyl-5-phosphono-3-pentensäure und ihrer pharmazeutisch annehmbaren Salze.

**21.** Verfahren gemäss einem der Ansprüche 1 bis 4 zur Herstellung von E-2-Amino-4-benzyl-5-phosphono-3-pentensäure und ihrer pharmazeutisch annehmbaren Salze.

**22.** Verfahren gemäss einem der Ansprüche 1 bis 4 zur Herstellung von E-2-Amino-4-methyl-5-phosphono-3-pentensäureethylester und seiner pharmazeutisch annehmbaren Salze.

**23.** Verfahren gemäss einem der Ansprüche 1 bis 4 zur Herstellung von E-2-Amino-4-methyl-5-phosphono-3-pentensäuremethylester und seiner pharmazeutisch annehmbaren Salze.

**24.** Verfahren gemäss einem der Ansprüche 1 bis 4 zur Herstellung von E-2-Amino-4-methyl-5-phosphono-3-pentensäure-n-propylester und seiner pharmazeutisch annehmbaren Salze.

**25.** Verfahren gemäss einem der Ansprüche 1 bis 4 zur Herstellung von E-2-Amino-4-methyl-5-phosphono-3-pentensäure-n-butylester und seiner pharmazeutisch annehmbaren Salze.

**26.** Verfahren gemäss einem der Ansprüche 1 bis 4 zur Herstellung von E-2-Amino-4-methyl-5-phosphono-3-pentensäure-iso-butylester und seiner pharmazeutisch annehmbaren Salze.

**27.** Verfahren gemäss einem der Ansprüche 1 bis 4 zur Herstellung von E-2-Amino-4-methyl-5-phosphono-3-pentensäure-n-pentylester und seiner pharmazeutisch annehmbaren Salze.

28. Verfahren gemäss einem der Ansprüche 1 bis 4 zur Herstellung von E-2-Amino-4-methyl-5-phosphono-3-pentensäure-n-hexylester und seiner pharmazeutisch annehmbaren Salze.

29. Verfahren zur Herstellung pharmazeutischer Präparate, dadurch gekennzeichnet, dass man eine gemäss einem der Ansprüche 1-28, erhältliche Verbindung mit einem pharmazeutisch geeigneten Trägermaterial vermischt.

**Claims**

**Claims for the following Contracting States : BE, CH, DE, ES, FR, GB, GR, IT, LI, LU, NL, SE**

1. A compound of the formula I

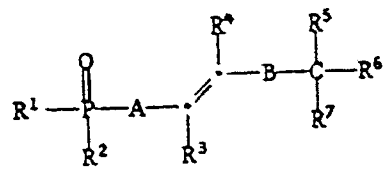

(I)

in which the carbon-carbon double bond is in the transconfiguration and $R^1$ represents hydroxy or etherified hydroxy, $R^2$ represents hydrogen, alkyl, hydroxy or etherified hydroxy, $R^3$ represents hydrogen, alkyl, haloalkyl, hydroxyalkyl, lower alkoxyalkyl, arylalkyl, lower alkenyl, halogen or aryl, $R^4$ represents hydrogen, alkyl or aryl, $R^5$ represents hydrogen or alkyl, $R^6$ represents carboxy, esterified carboxy or amidated carboxy, $R^7$ represents amino or amino substituted by alkyl or by acyl, A represents unsubstituted or alkylsubstituted $\alpha,\omega$-alkylene having from 1 to 3 carbon atoms, B represents a bond, and "lower" radicals contain up to and including 8 carbon atoms, or a salt thereof.

2. A compound of the formula I according to claim 1 in which $R^3$ represents hydrogen, alkyl or aryl.

3. A compound of the formula I according to claim 1 in which $R^6$ represents lower alkoxycarbonyl or phenyl-lower alkoxycarbonyl.

4. A compound of the formula I according to claim 1 in which $R^1$ represents hydroxy, lower alkoxy or hydroxy-lower alkoxy, $R^2$ represents hydrogen, alkyl, hydroxy, lower alkoxy or hydroxy-lower alkoxy, $R^3$ represents hydrogen, lower alkyl, halo-lower alkyl, hydroxy-lower alkyl, lower alkoxy-lower alkyl, phenyl-lower alkyl that is unsubstituted or substituted in the phenyl moiety, lower alkenyl, halogen, or unsubstituted or substituted phenyl, $R^4$ represents hydrogen, lower alkyl or unsubstituted or substituted phenyl, $R^5$ represents hydrogen or lower alkyl, $R^6$ represents carboxy or pharmaceutically acceptable esterified or amidated carboxy, $R^7$ represents amino, mono- or di-lower alkylamino, alkanoylamino or alkanoylamino substituted by halogen, by amino and/or by phenyl, carbamoyl, carboxy, imidazolyl, lower alkylthio, tetrahydropyrrolyl, hydroxy, indolyl or by hydroxyphenyl, benzoylamino or benzoylamino substituted by halogen, lower alkoxy or by nitro, or phthalimino, A represents unsubstituted or lower alkyl-substituted $\alpha,\omega$-alkylene having from 1 to 3 carbon atoms, and B represents a bond, wherein the substituents of phenyl are selected from the group consisting of lower alkyl, hydroxy, lower alkoxy, halogen, amino, halo-lower alkyl, hydroxy-lower alkyl, amino-lower alkyl and nitro, or a pharmaceutically acceptable salt thereof.

5. A compound of the formula I according to claim 3 in which $R^6$ represents carboxy, alkoxycarbonyl, or alkoxycarbonyl substituted by amino, mono- or di-lower alkylamino, hydroxy or by lower alkanoyloxy, $R^7$ represents amino, mono-lower alkylamino, lower alkanoylamino or benzoylamino, or a pharmaceutically acceptable salt thereof.

6. A compound of the formula I according to claim 4 in which $R^3$ and $R^4$, independently of one another, each represents hydrogen, lower alkyl, phenyl, or phenyl substituted by lower alkyl, hydroxy, lower alkoxy, halogen, amino, halo-lower alkyl, hydroxy-lower alkyl, amino-lower alkyl or by nitro, or a pharmaceutically acceptable salt thereof.

7. A compound of the formula I according to claim 1 in which $R^1$ represents hydroxy or lower alkoxy, $R^2$

represents hydrogen, alkyl, hydroxy or lower alkoxy, $R^3$ represents hydrogen, lower alkyl, phenyl, halophenyl or phenyl-lower alkyl, $R^4$ and $R^5$ represent hydrogen or lower alkyl, $R^6$ represents carboxy, alkoxycarbonyl or hydroxy-lower alkoxycarbonyl, $R^7$ represents amino, mono-lower alkylamino, lower alkanoylamino or benzoylamino, A represents unsubstituted or lower alkyl-substituted $\alpha,\omega$-alkylene having from 1 to 3 carbon atoms, and B represents a bond, or a pharmaceutically acceptable salt thereof.

**8.** A compound of the formula I according to claim 1 in which $R^1$ is hydroxy, $R^2$ represents hydrogen, alkyl or hydroxy, $R^3$ represents hydrogen, lower alkyl or halophenyl, $R^4$ represents hydrogen or halophenyl and $R^5$ represents hydrogen, $R^6$ represents carboxy, lower alkoxycarbonyl or hydroxy-lower alkoxycarbonyl, $R^7$ represents amino, mono-lower alkylamino, lower alkanoylamino or benzoylamino, A represents $\alpha,\omega$-alkylene having from 1 to 3 carbon atoms, and B represents a bond, or a pharmaceutically acceptable salt thereof.

**9.** A compound of the formula I according to claim 1 in which $R^1$ is hydroxy, $R^2$ represents hydrogen, lower alkyl or hydroxy, $R^3$ represents hydrogen or lower alkyl, $R^4$ and $R^5$ represent hydrogen, $R^6$ represents carboxy or lower alkoxycarbonyl, $R^7$ represents amino or mono-lower alkylamino, A represents $\alpha,\omega$-alkylene having from 1 to 3 carbon atoms, and B represents a bond, or a pharmaceutically acceptable salt thereof.

**10.** A compound of the formula I according to claim 1 in which $R^1$ and $R^2$ represent hydroxy, $R^3$ represents hydrogen or lower alkyl, $R^4$ and $R^5$ represent hydrogen, $R^6$ represents carboxy, $R^7$ represents amino, A represents methylene and B represents a bond, or a carboxylic acid lower alkyl ester or a pharmaceutically acceptable salt thereof.

**11.** E-2-amino-5-phosphono-3-pentenoic acid or a pharmaceutically acceptable salt thereof.

**12.** E-2-amino-4-methyl-5-phosphono-3-pentenoic acid or a pharmaceutically acceptable salt thereof.

**13.** (D)-E-2-amino-4-methyl-5-phosphono-3-pentenoic acid or a pharmaceutically acceptable salt thereof.

**14.** E-2-amino-4-ethyl-5-phosphono-3-pentenoic acid or a pharmaceutically acceptable salt thereof.

**15.** E-2-amino-4-propyl-5-phosphono-3-pentenoic acid or a pharmaceutically acceptable salt thereof.

**16.** E-2-amino-4-butyl-5-phosphono-3-pentenoic acid or a pharmaceutically acceptable salt thereof.

**17.** E-2-amino-4-isopropyl-5-phosphono-3-pentenoic acid or a pharmaceutically acceptable salt thereof.

**18.** E-2-amino-4-benzyl-5-phosphono-3-pentenoic acid or a pharmaceutically acceptable salt thereof.

**19.** E-2-amino-4-methyl-5-phosphono-3-pentenoic acid ethyl ester or a salt thereof.

**20.** E-2-amino-4-methyl-5-phosphono-3-pentenoic acid methyl ester or a salt thereof.

**21.** E-2-amino-4-methyl-5-phosphono-3-pentenoic acid n-propyl ester or a salt thereof.

**22.** E-2-amino-4-methyl-5-phosphono-3-pentenoic acid n-butyl ester or a salt thereof.

**23.** E-2-amino-4-methyl-5-phosphono-3-pentenoic acid isobutyl ester or a salt thereof.

**24.** E-2-amino-4-methyl-5-phosphono-3-pentenoic acid n-pentyl ester or a salt thereof.

**25.** E-2-amino-4-methyl-5-phosphono-3-pentenoic acid n-hexyl ester or a salt thereof.

**26.** A pharmaceutical preparation comprising a compound of claim 3, 6, 8, 11 or 12 together with a pharmaceutically suitable carrier material.

**27.** A pharmaceutical preparation comprising a compound of claim 1, 4, 5, 7, 9, 10 or 13 to 25 together with a pharmaceutically suitable carrier material.

**28.** A compound according to claim 1 for use in a method of therapeutically treating the human or animal body.

**29.** A compound according to claim 1 as an anti-convulsive agent.

**30.** The use of a compound according to claim 1 of a pharmaceutical preparation for use as an anti-convulsive agent.

**31.** A process for the manufacture of a compound of the formula I claimed in claim 1, or a salt thereof, characterised in that

a) a compound of the formula II

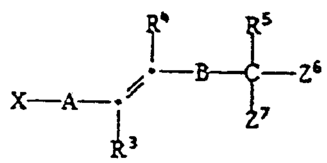

(II)

in which $R^3$, $R^4$, $R^5$, A and B are as defined for formula I, $Z^6$ has the meaning of $R^6$ or represents protected carboxy, $Z^7$ has the meaning of $R^7$ or represents protected amino and X represents reactive esterified hydroxy, is reacted with a compound of the formula III

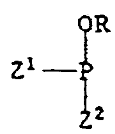

(III)

in which $Z^1$ has the meaning of $R^1$ or represents protected hydroxy, $Z^2$ has the meaning of $R^2$ or represents protected hydrogen or protected hydroxy, and R represents an etherifying group, or

b) in order to obtain a compound of the formula I in which $R^5$ represents hydrogen, in a compound of the formula IV

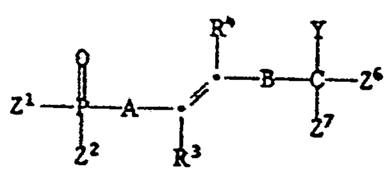

(IV)

in which $R^3$, $R^4$, A and B are as defined for formula I, $Z^1$ has the meaning of $R^1$ or represents protected hydroxy, $Z^2$ has the meaning of $R^2$ or represents protected hydroxy or protected hydrogen, $Z^5$ has the meaning of $R^6$ or represents protected carboxy, $Z^7$ has the meaning of $R^7$ or represents protected amino, and Y represents an optionally esterified carboxy group that can be replaced by hydrogen, the group Y is replaced by hydrogen, and any protected functional groups that may be present in a compound resulting from one of the preceding processes are freed and, if desired, a resulting compound of the formula I is converted into a different compound of the formula I and/or, if desired, a resulting compound of the formula I is converted into a salt or a resulting salt is converted into a different salt or into a free compound of the formula I and/or, if desired, an optical isomer is isolated from a mixture of stereoisomeric forms of a resulting compound of the formula I or of a salt thereof.

**32.** A process according to claim 31, characterised in that a compound of the formula II

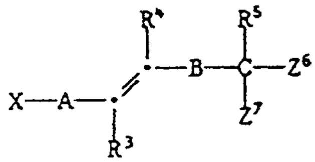

(II)

in which $R^3$, $R^4$, $R^5$, A and B are as defined for formula I, $Z^6$ represents protected carboxy, $Z^7$ represents protected amino and X represents reactive esterified hydroxy, is reacted with a compound of the formula III

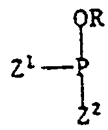

(III)

in which $Z^1$ represents protected hydroxy, $Z^2$ represents lower alkyl, protected hydrogen or protected hydroxy and R represents lower alkyl, and following this reaction the protected groups are freed.

**33.** A process according to claim 32, in which $Z^1$ represents lower alkoxy, $Z^2$ represents lower alkyl, di-lower alkoxy-lower alkyl or lower alkoxy, R represents lower alkyl, $Z^6$ represents lower alkoxycarbonyl, $Z^7$ represents formylamino and X represents halogen.

**34.** A process according to claim 31 for the conversion of a compound of the formula I in which $R^6$ represents carboxy into a compound of the formula I in which $R^6$ represents esterified carboxy.

**Claims for the following Contracting State : AT, ES, GR**

**1.** A process for the manufacture of a compound of the formula I

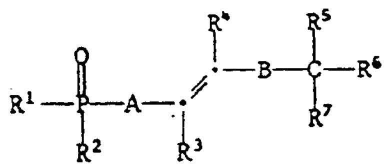

(I)

in which the carbon-carbon double bond is in the transconfiguration and $R^1$ represents hydroxy or etherified hydroxy, $R^2$ represents hydrogen, alkyl, hydroxy or etherified hydroxy, $R^3$ represents hydrogen, alkyl, haloalkyl, hydroxyalkyl, lower alkoxyalkyl, arylalkyl, lower alkenyl, halogen or aryl, $R^4$ represents hydrogen, alkyl or aryl, $R^5$ represents hydrogen or alkyl, $R^6$ represents carboxy, esterified carboxy or amidated carboxy, $R^7$ represents amino or amino substituted by alkyl or by acyl, A represents unsubstituted or alkylsubstituted $\alpha,\omega$-alkylene having from 1 to 3 carbon atoms, B represents a bond, and "lower" radicals contain up to and including 8 carbon atoms, or a salt thereof, characterised in that

a) a compound of the formula II

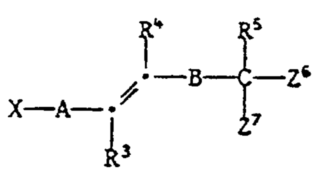

(II)

in which $R^3$, $R^4$, $R^5$, A and B are as defined for formula I, $Z^6$ has the meaning of $R^6$ or represents protected carboxy, $Z^7$ has the meaning of $R^7$ or represents protected amino and X represents reactive esterified hydroxy, is reacted with a compound of the formula III

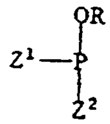

(III)

in which $Z^1$ has the meaning of $R^1$ or represents protected hydroxy, $Z^2$ has the meaning of $R^2$ or represents protected hydrogen or protected hydroxy, and R represents an etherifying group, or

b) in order to obtain a compound of the formula I in which $R^5$ represents hydrogen, in a compound of the formula IV

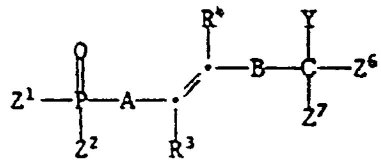

(IV)

in which $R^3$, $R^4$, A and B are as defined for formula I, $Z^1$ has the meaning of $R^1$ or represents protected hydroxy, $Z^2$ has the meaning of $R^2$ or represents protected hydroxy or protected hydrogen, $Z^6$ has the meaning of $R^6$ or represents protected carboxy, $Z^7$ has the meaning of $R^7$ or represents protected amino, and Y represents an optionally esterified carboxy group that can be replaced by hydrogen, the group Y is replaced by hydrogen, and any protected functional groups that may be present in a compound resulting from one of the preceding processes are freed and, if desired, a resulting compound of the formula I is converted into a different compound of the formula I and/or, if desired, a resulting compound of the formula I is converted into a salt or a resulting salt is converted into a different salt or into a free compound of the formula I and/or, if desired, an optical isomer is isolated from a mixture of stereoisomeric forms of a resulting compound of the formula I or of a salt thereof.

**2.** A process according to claim 1, characterised in that a compound of the formula II

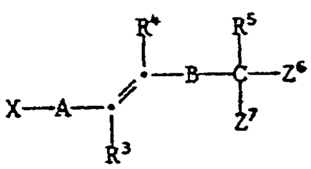

(II)

in which $R^3$, $R^4$, $R^5$, A and B are as defined for formula I, $Z^6$ represents protected carboxy, $Z^7$ represents protected amino and X represents reactive esterified hydroxy, is reacted with a compound of the formula III

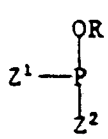

(III)

in which $Z^1$ represents protected hydroxy, $Z^2$ represents lower alkyl, protected hydrogen or protected hydroxy and R represents lower alkyl, and following this reaction the protected groups are freed.

**3.** A process according to claim 2, in which $Z^1$ represents lower alkoxy, $Z^2$ represents lower alkyl, di-lower alkoxy-lower alkyl or lower alkoxy, R represents lower alkyl, $Z^6$ represents lower alkoxycarbonyl, $Z^7$ represents formylamino and X represents halogen.

**4.** A process according to claim 1 for the conversion of a compound of the formula I in which $R^6$

represents carboxy into a compound of the formula I in which $R^6$ represents esterified carboxy.

5. A process according to any one of claims 1 to 4 for the manufacture of a compound of the formula I according to claim 1 in which $R^3$ represents hydrogen, alkyl or aryl.

6. A process according to any one of claims 1 to 4 for the manufacture of a compound of the formula I according to claim 1 in which $R^6$ represents lower alkoxycarbonyl or phenyl-lower alkoxycarbonyl.

7. A process according to any one of claims 1 to 4 for the manufacture of a compound of the formula I in which $R^1$ represents hydroxy, lower alkoxy or hydroxy-lower alkoxy, $R^2$ represents hydrogen, alkyl, hydroxy, lower alkoxy or hydroxy-lower alkoxy, $R^3$ represents hydrogen, lower alkyl, halo-lower alkyl, hydroxy-lower alkyl, lower alkoxy-lower alkyl, phenyl-lower alkyl that is unsubstituted or substituted in the phenyl moiety, lower alkenyl, halogen, or unsubstituted or substituted phenyl, $R^4$ represents hydrogen, lower alkyl or unsubstituted or substituted phenyl, $R^5$ represents hydrogen or lower alkyl, $R^6$ represents carboxy or pharmaceutically acceptable esterified or amidated carboxy, $R^7$ represents amino, mono- or di-lower alkylamino, alkanoylamino or alkanoylamino substituted by halogen, by amino and/or by phenyl, carbamoyl, carboxy, imidazolyl, lower alkylthio, tetrahydropyrrolyl, hydroxy, indolyl or by hydroxyphenyl, benzoylamino or benzoylamino substituted by halogen, lower alkoxy or by nitro, or phthalimino, A represents unsubstituted or lower alkyl-substituted $\alpha,\omega$-alkylene having from 1 to 3 carbon atoms, and B represents a bond, wherein the substituents of phenyl are selected from the group consisting of lower alkyl, hydroxy, lower alkoxy, halogen, amino, halo-lower alkyl, hydroxy-lower alkyl, amino-lower alkyl and nitro, or a pharmaceutically acceptable salt thereof.

8. A process according to claim 7 for the manufacture of a compound of the formula I in which $R^6$ represents carboxy, alkoxycarbonyl, or alkoxycarbonyl substituted by amino, mono- or di-lower alkylamino, hydroxy or by lower alkanoyloxy, $R^7$ represents amino, mono-lower alkylamino, lower alkanoylamino or benzoylamino, or a pharmaceutically acceptable salt thereof.

9. A process according to claim 8 for the manufacture of a compound of the formula I in which $R^3$ and $R^4$, independently of one another, each represents hydrogen, lower alkyl, phenyl, or phenyl substituted by lower alkyl, hydroxy, lower alkoxy, halogen, amino, halo-lower alkyl, hydroxy-lower alkyl, amino-lower alkyl or by nitro, or a pharmaceutically acceptable salt thereof.

10. A process according to any one of claims 1 to 4 for the manufacture of a compound of the formula I in which $R^1$ represents hydroxy or lower alkoxy, $R^2$ represents hydrogen, alkyl, hydroxy or lower alkoxy, $R^3$ represents hydrogen, lower alkyl, phenyl, halophenyl or phenyl-lower alkyl, $R^4$ and $R^5$ represent hydrogen or lower alkyl, $R^6$ represents carboxy, alkoxycarbonyl or hydroxy-lower alkoxycarbonyl, $R^7$ represents amino, mono-lower alkylamino, lower alkanoylamino or benzoylamino, A represents unsubstituted or lower alkyl-substituted $\alpha,\omega$-alkylene having from 1 to 3 carbon atoms, and B represents a bond, or a pharmaceutically acceptable salt thereof.

11. A process according to any one of claims 1 to 4 for the manufacture of a compound of the formula I in which $R^1$ is hydroxy, $R^2$ represents hydrogen, alkyl or hydroxy, $R^3$ represents hydrogen, lower alkyl or halophenyl, $R^4$ represents hydrogen or halophenyl and $R^5$ represents hydrogen, $R^6$ represents carboxy, lower alkoxycarbonyl or hydroxy-lower alkoxycarbonyl, $R^7$ represents amino, mono-lower alkylamino, lower alkanoylamino or benzoylamino, A represents $\alpha,\omega$-alkylene having from 1 to 3 carbon atoms, and B represents a bond, or a pharmaceutically acceptable salt thereof.

12. A process according to any one of claims 1 to 4 for the manufacture of a compound of the formula I in which $R^1$ is hydroxy, $R^2$ represents hydrogen, lower alkyl or hydroxy, $R^3$ represents hydrogen or lower alkyl, $R^4$ and $R^5$ represent hydrogen, $R^6$ represents carboxy or lower alkoxycarbonyl, $R^7$ represents amino or mono-lower alkylamino, A represents $\alpha,\omega$-alkylene having from 1 to 3 carbon atoms, and B represents a bond, or a pharmaceutically acceptable salt thereof.

13. A compound of the formula I according to claim 1 in which $R^1$ and $R^2$ represent hydroxy, $R^3$ represents hydrogen or lower alkyl, $R^4$ and $R^5$ represent hydrogen, $R^6$ represents carboxy, $R^7$ represents amino, A represents methylene and B represents a bond, or a carboxylic acid lower alkyl ester or a pharmaceutically acceptable salt thereof.

14. A process according to any one of claims 1 to 4 for the manufacture of E-2-amino-5-phosphono-3-pentenoic acid or a pharmaceutically acceptable salt thereof.

15. A process according to any one of claims 1 to 4 for the manufacture of E-2-amino-4-methyl-5-phosphono-3-pentenoic acid or a pharmaceutically acceptable salt thereof.

16. A process according to any one of claims 1 to 4 for the manufacture of (D)-E-2-amino-4-methyl-5-phosphono-3-pentenoic acid or a pharmaceutically acceptable salt thereof.

17. A process according to any one of claims 1 to 4 for the manufacture of E-2-amino-4-ethyl-5-phosphono-3-pentenoic acid or a pharmaceutically acceptable salt thereof.

18. A process according to any one of claims 1 to 4 for the manufacture of E-2-amino-4-propyl-5-phosphono-3-pentenoic acid or a pharmaceutically acceptable salt thereof.

19. A process according to any one of claims 1 to 4 for the manufacture of E-2-amino-4-butyl-5-phosphono-3-pentenoic acid or a pharmaceutically acceptable salt thereof.

20. A process according to any one of claims 1 to 4 for the manufacture of E-2-amino-4-isopropyl-5-phosphono-3-pentenoic acid or a pharmaceutically acceptable salt thereof.

21. A process according to any one of claims 1 to 4 for the manufacture of E-2-amino-4-benzyl-5-phosphono-3-pentenoic acid or a pharmaceutically acceptable salt thereof.

22. A process according to any one of claims 1 to 4 for the manufacture of E-2-amino-4-methyl-5-phosphono-3-pentenoic acid ethyl ester or a pharmaceutically acceptable salt thereof.

23. A process according to any one of claims 1 to 4 for the manufacture of E-2-amino-4-methyl-5-phosphono-3-pentenoic acid methyl ester or a pharmaceutically acceptable salt thereof.

24. A process according to any one of claims 1 to 4 for the manufacture of E-2-amino-4-methyl-5-phosphono-3-pentenoic acid n-propyl ester or a pharmaceutically acceptable salt thereof.

25. A process according to any one of claims 1 to 4 for the manufacture of E-2-amino-4-methyl-5-phosphono-3-pentenoic acid n-butyl ester or a pharmaceutically acceptable salt thereof.

26. A process according to any one of claims 1 to 4 for the manufacture of E-2-amino-4-methyl-5-phosphono-3-pentenoic acid isobutyl ester or a pharmaceutically acceptable salt thereof.

27. A process according to any one of claims 1 to 4 for the manufacture of E-2-amino-4-methyl-5-phosphono-3-pentenoic acid n-pentyl ester or a pharmaceutically acceptable salt thereof.

28. A process according to any one of claims 1 to 4 for the manufacture of E-2-amino-4-methyl-5-phosphono-3-pentenoic acid n-hexyl ester or a pharmaceutically acceptable salt thereof.

29. A process for the manufacture of a pharmaceutical preparation, characterised in that a compound obtainable according to any one of claims 1 to 28 is mixed with a pharmaceutically suitable carrier material.

**Revendications**
**Revendications pour les Etats contractants suivants : BE, CH, DE, ES, FR, GB, GR, IT, LI, LU, NL, SE**

1. Composés de formule I

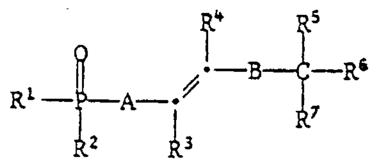

(I)

dans laquelle la double liaison carbone-carbone est en configuration trans et $R^1$ représente un groupe hydroxy ou hydroxy éthérifié, $R^2$ représente l'hydrogène, un groupe alkyle, hydroxy ou hydroxy éthérifié, $R^3$ représente l'hydrogène, un groupe alkyle, halogénoalkyle, hydroxyalkyle, (alcoxy inférieur)-alkyle, arylalkyle, alcényle inférieur, un halogène ou un groupe aryle, $R^4$ représente l'hydrogène, un groupe alkyle ou aryle, $R^5$ représente l'hydrogène ou un groupe alkyle, $R^6$ représente un groupe carboxy, carboxy estérifié ou amidé, $R^7$ représente un groupe amino ou amino à substituants alkyle ou acyle, A représente un groupe alpha,oméga-alkylène en C 1-C 3 non substitué ou substitué par un groupe alkyle, B représente une liaison et les groupes qualifiés d'"inférieurs" contiennent jusqu'à 8 atomes de carbone inclus, et leurs sels.

2. Composés de formule I de la revendication 1, dans lesquels $R^3$ représente l'hydrogène, un groupe alkyle ou aryle.

3. Composés de formule I de la revendication 1, dans lesquels $R^6$ représente un groupe (alcoxy inférieur)-ou phényl-(alcoxy inférieur)-carbonyle.

4. Composés de formule I de la revendication 1, dans lesquels $R^1$ représente un groupe hydroxy, alcoxy inférieur ou hydroxyalcoxy inférieur, $R^2$ représente l'hydrogène, un groupe alkyle, hydroxy, alcoxy inférieur ou hydroxyalcoxy inférieur, $R^3$ représente l'hydrogène, un groupe alkyle inférieur, halogénoalkyle inférieur, hydroxyalkyle inférieur, (alcoxy inférieur)-alkyle inférieur, phényl-alkyle inférieur non substitué ou substitué dans la partie phényle, alcényle inférieur, un halogène ou un groupe phényle non substitué ou substitué, $R^4$ représente l'hydrogène, un groupe alkyle inférieur ou un groupe phényle non substitué ou substitué, $R^5$ représente l'hydrogène ou un groupe alkyle inférieur, $R^6$ représente un groupe carboxy ou un groupe carboxy estérifié ou amidé acceptable pour l'usage pharmaceutique, $R^7$ représente un groupe amino, mono- ou di-(alkyle inférieur)-amino, alcanoylamino ou un groupe alcanoylamino substitué par des halogènes, des groupes amino et/ou phényle, carbamoyle, carboxy, imidazolyle, alkylthio inférieur, tétrahydropyrrolyle, hydroxy, indolyle ou hydroxyphényle, un groupe benzoylamino ou un groupe benzoylamino ou phtalimino substitué par des halogènes, des groupes alcoxy inférieurs ou nitro, A représente un groupe alpha,oméga-alkylène en C 1-C 3 non substitué ou substitué par un groupe alkyle inférieur et B représente une liaison, les substituants des groupes phényle étant choisis parmi les groupes alkyle inférieurs, hydroxy, alcoxy inférieurs, les halogènes, les groupes amino, halogénoalkyle inférieurs, hydroxyalkyle inférieurs, aminoalkyle inférieurs et nitro, et leurs sels acceptables pour l'usage pharmaceutique.

5. Composés de formule I de la revendication 3, dans lesquels $R^6$ représente un groupe carboxy, alcoxycarbonyle ou alcoxycarbonyle substitué par des groupes amino, mono- ou di-(alkyle inférieur)-amino, hydroxy ou alcanoyloxy inférieurs, $R^7$ représente un groupe amino, mono-(alkyle inférieur)-amino, alcanoylamino inférieur ou benzoylamino, et leurs sels acceptables pour l'usage pharmaceutique.

6. Composés de formule I de la revendication 4, dans lesquels $R^3$ et $R^4$ représentent chacun, indépendamment l'un de l'autre, l'hydrogène, un groupe alkyle inférieur, phényle ou phényle substitué par des groupes alkyle inférieurs, hydroxy, alcoxy inférieurs, des halogènes, des groupes amino, halogénoalkyle inférieurs, hydroxyalkyle inférieurs, aminoalkyle inférieurs ou nitro, et leurs sels acceptables pour l'usage pharmaceutique.

7. Composés de formule I de la revendication 1, dans lesquels $R^1$ représente un groupe hydroxy ou alcoxy inférieur, $R^2$ représente l'hydrogène, un groupe alkyle, hydroxy ou alcoxy inférieur, $R^3$ représente l'hydrogène, un groupe alkyle inférieur, phényle, halogénophényle ou phényl-alkyle inférieur, $R^4$ et $R^5$ représentent l'hydrogène ou des groupes alkyle inférieurs, $R^6$ représente un groupe carboxy, alcoxy-carbonyle ou hydroxy-(alcoxy inférieur)-carbonyle, $R^7$ représente un groupe amino, mono-(alkyle

inférieur)-amino, alcanoylamino inférieur ou benzoylamino, A représente un groupe alpha,oméga-alkylène en C 1-C 3 non substitué ou substitué par un groupe alkyle inférieur et B représente une liaison, et leurs sels acceptables pour l'usage pharmaceutique.

8. Composés de formule I de la revendication 1, dans lesquels $R^1$ représente un groupe hydroxy, $R^2$ représente l'hydrogène, un groupe alkyle ou hydroxy, $R^3$ représente l'hydrogène, un groupe alkyle inférieur ou halogénophényle, $R^4$ représente l'hydrogène ou un groupe halogénophényle et $R^5$ représente l'hydrogène, $R^6$ représente un groupe carboxy, (alcoxy inférieur)-carbonyle ou hydroxy-(alcoxy inférieur)-carbonyle, $R^7$ représente un groupe amino, mono-(alkyle inférieur)-amino, alcanoylamino inférieur ou benzoylamino, A représente un groupe alpha, oméga-alkylène en C 1-C 3 et B une liaison, et leurs sels acceptables pour l'usage pharmaceutique.

9. Composés de formule I de la revendication 1, dans lesquels $R^1$ représente un groupe hydroxy, $R^2$ représente l'hydrogène, un groupe alkyle inférieur ou hydroxy, $R^3$ représente l'hydrogène ou un groupe alkyle inférieur, $R^4$ et $R^5$ représentent l'hydrogène, $R^6$ représente un groupe carboxy ou (alcoxy inférieur)-carbonyle, $R^7$ représente un groupe amino ou mono-(alkyle inférieur)-amino, A représente un groupe alpha,oméga-alkylène en C 1-C 3 et B une liaison, et leurs sels acceptables pour l'usage pharmaceutique.

10. Composés de formule I de la revendication 1, dans lesquels $R^1$ et $R^2$ représentent des groupes hydroxy, $R^3$ l'hydrogène ou un groupe alkyle inférieur, $R^4$ et $R^5$ l'hydrogène, $R^6$ un groupe carboxy, $R^7$ un groupe amino, A un groupe méthylène et B une liaison, leurs esters alkyliques inférieurs d'acides carboxyliques et leurs sels acceptables pour l'usage pharmaceutique.

11. L'acide E-2-amino-5-phosphono-3-pent énoïque et ses sels acceptables pour l'usage pharmaceutique.

12. L'acide E-2-amino-4-méthyl-5-phosphono-3-penténoïque et ses sels acceptables pour l'usage pharmaceutique.

13. L'acide (D)-E-2-amino-4-méthyl-5-phosphono-3-penténoïque et ses sels acceptables pour l'usage pharmaceutique.

14. L'acide E-2-amino-4-éthyl-5-phosphono-3-penténoïque et ses sels acceptables pour l'usage pharmaceutique.

15. L'acide E-2-amino-4-propyl-5-phosphono-3-penténoïque et ses sels acceptables pour l'usage pharmaceutique.

16. L'acide E-2-amino-4-butyl-5-phosphono-3-penténoïque et ses sels acceptables pour l'usage pharmaceutique.

17. L'acide E-2-amino-4-isopropyl-5-phosphono-3-penténoïque et ses sels acceptables pour l'usage pharmaceutique.

18. L'acide E-2-amino-4-benzyl-5-phosphono-3-penténoïque et ses sels acceptables pour l'usage pharmaceutique.

19. Le E-2-amino-4-méthyl-5-phosphono-3-penténoate d'éthyle ou l'un de ses sels.

20. Le E-2-amino-4-méthyl-5-phosphono-3-penténoate de méthyle ou l'un de ses sels.

21. Le E-2-amino-4-méthyl-5-phosphono-3-penténoate de n-propyle ou l'un de ses sels.

22. Le E-2-amino-4-méthyl-5-phosphono-3-penténoate de n-butyle ou l'un de ses sels.

23. Le E-2-amino-4-méthyl-5-phosphono-3-penténoate d'isobutyle ou l'un de ses sels.

24. Le E-2-amino-4-méthyl-5-phosphono-3-penténoate de n-pentyle ou l'un de ses sels.

**25.** Le E-2-amino-4-méthyl-5-phosphono-3-penténoate de n-hexyle ou l'un de ses sels.

**26.** Compositions pharmaceutiques contenant des composés des revendications 3, 6, 8, 11 et 12, avec un véhicule acceptable pour l'usage pharmaceutique.

**27.** Compositions pharmaceutiques contenant des composés de revendications 1, 4, 5, 7, 9, 10 et 13 à 25, avec un véhicule acceptable pour l'usage pharmaceutique.

**28.** Composés selon revendication 1, pour l'utilisation dans un procédé pour le traitement thérapeutique de l'organisme humain ou animal.

**29.** Les composés selon revendication 1 en tant qu'agents anticonvulsifs.

**30.** Utilisation des composés selon revendication 1 pour la préparation de compositions pharmaceutiques servant de médicaments anticonvulsifs.

**31.** Procédé de préparation des composés de formule I de la revendication 1 et de leurs sels, caractérisé en ce que :

a) on fait réagir un composé de formule II

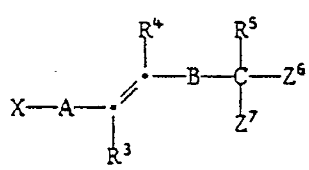

(II)

dans laquelle $R^3$, $R^4$, $R^5$, A et B ont les significations indiquées en référence à la formule I, $Z^6$ a les significations de $R^6$ ou représente un groupe carboxy protégé, $Z^7$ a les significations de $R^7$ ou représente un groupe amino protégé et X représente un groupe hydroxy estérifié réactif, avec un composé de formule III

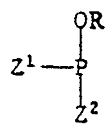

(III)

dans laquelle $Z^1$ a les significations de $R^1$ ou représente un groupe hydroxy protégé, $Z^2$ a les significations de $R^2$ ou représente l'hydrogène protégé ou un groupe hydroxy protégé et R représente un groupe éthérifiant, ou bien

b) pour obtenir un composé de formule I dans laquelle $R^5$ représente l'hydrogène, partant d'un composé de formule IV

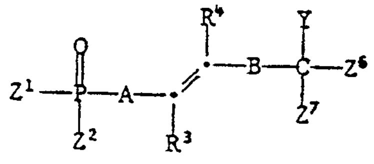

(IV)

dans laquelle $R^3$, $R^4$, A et B ont les significations indiquées en référence à la formule I, $Z^1$ a les significations de $R^1$ ou représente un groupe hydroxy protégé, $Z^2$ a les significations de $R^2$ ou représente un groupe hydroxy protégé ou l'hydrogène protégé, $Z^6$ a les significations de $R^6$ ou représente un groupe carboxy protégé, $Z^7$ a les significations de $R^7$ ou représente un groupe amino protégé, et Y représente un groupe carboxy éventuellement estérifié remplaçable par l'hydrogène, on remplace le groupe Y par l'hydrogène, et dans un composé obtenu par l'un des procédés ci-dessus, on libère le cas échéant les groupes fonctionnels protégés, et si on le désire, on convertit

un composé de formule I obtenu par l'un de ces procédés en un autre composé de formule I, et/ou si on le désire, on convertit un composé obtenu, répondant à la formule I, en un sel ou un sel obtenu en un autre sel ou en un composé de formule I à l'état libre, et/ou si on le désire, on isole un isomère optique à partir d'un mélange de formes stéréoisomères d'un composé de formule I ainsi obtenu ou de l'un de ses sels.

**32.** Procédé selon revendication 31, caractérisé en ce que l'on fait réagir un composé de formule II

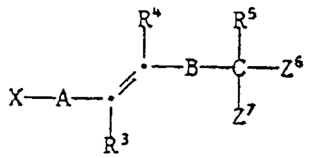

(II)

dans laquelle $R^3$, $R^4$, $R^5$, A et B ont les significations indiquées en référence à la formule I, $Z^6$ représente un groupe carboxy protégé, $Z^7$ un groupe amino protégé et X un groupe hydroxy estérifié réactif, avec un composé de formule III

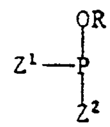

(III)

dans laquelle $Z^1$ représente un groupe hydroxy protégé, $Z^2$ représente un groupe alkyle inférieur, l'hydrogène protégé ou un groupe hydroxy protégé et R un groupe alkyle inférieur, et on libère ensuite les groupes protégés.

**33.** Procédé selon revendication 32, dans lequel $Z^1$ représente un groupe alcoxy inférieur, $Z^2$ un groupe alkyle inférieur, di-(alcoxy inférieur)-alkyle inférieur ou alcoxy inférieur, R un groupe alkyle inférieur, $Z^6$ un groupe (alcoxy inférieur)-carbonyle, $Z^7$ un groupe formylamino et X un halogène.

**34.** Procédé selon revendication 31, pour convertir un composé de formule I dans laquelle $R^6$ représente un groupe carboxy en un composé de formule I dans laquelle $R^6$ représente un groupe carboxy estérifié.

**Revendications pour l'Etat contractant suivant : AT, ES, GR**

**1.** Procédé de préparation de composés de formule I

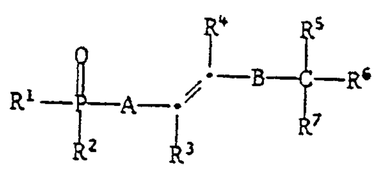

(I)

dans laquelle la double liaison carbone-carbone est en configuration trans et $R^1$ représente un groupe hydroxy ou hydroxy éthérifié, $R^2$ représente l'hydrogène, un groupe alkyle, hydroxy ou hydroxy éthérifié, $R^3$ représente l'hydrogène, un groupe alkyle, halogénoalkyle, hydroxyalkyle, (alcoxy inférieur)-alkyle, arylalkyle, alcényle inférieur, un halogène ou un groupe aryle, $R^4$ représente l'hydrogène, un groupe alkyle ou aryle, $R^5$ représente l'hydrogène ou un groupe alkyle, $R^6$ représente un groupe carboxy, carboxy estérifié ou amidé, $R^7$ représente un groupe amino ou amino à substituants alkyle ou acyle, A représente un groupe alpha,oméga-alkylène en C 1-C 3 non substitué ou substitué par un groupe alkyle, B représente une liaison et les groupes qualifiés d'"inférieurs" contiennent jusqu'à 8 atomes de carbone inclus, et de leurs sels, caractérisé en ce que :

a) on fait réagir un composé de formule II

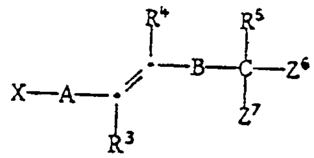

(II)

dans laquelle $R^3$, $R^4$, $R^5$, A et B ont les significations indiquées en référence à la formule I, $Z^6$ a les significations de $R^6$ ou représente un groupe carboxy protégé, $Z^7$ a les significations de $R^7$ ou représente un groupe amino protégé et X représente un groupe hydroxy estérifié réactif, avec un composé de formule III

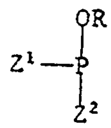

(III)

dans laquelle $Z^1$ a les significations de $R^1$ ou représente un groupe hydroxy protégé, $Z^2$ a les significations de $R^2$ ou représente l'hydrogène protégé ou un groupe hydroxy protégé et R représente un groupe éthérifiant, ou bien

b) pour obtenir un composé de formule I dans laquelle $R^5$ représente l'hydrogène, partant d'un composé de formule IV

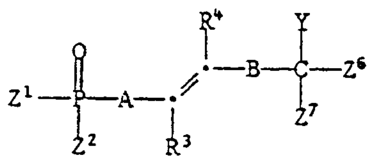

(IV)

dans laquelle $R^3$, $R^4$, $R^5$, A et B ont les significations indiquées en référence à la formule I, $Z^1$ a les significations de $R^1$ ou représente un groupe hydroxy protégé, $Z^2$ a les significations de $R^2$ ou représente un groupe hydroxy protégé ou l'hydrogène protégé, $Z^6$ a les significations de $R^6$ ou représente un groupe carboxy protégé, $Z^7$ a les significations de $R^7$ ou représente un groupe amino protégé et Y représente un groupe carboxy éventuellement estérifié remplaçable par l'hydrogène, on remplace le groupe Y par l'hydrogène, et dans un composé obtenu par l'un des procédés ci-dessus, on libère le cas échéant les groupes fonctionnels protégés, et si on le désire, on convertit un composé obtenu répondant à la formule I en un autre composé de formule I et/ou si on le désire, on convertit un composé obtenu, répondant à la formule I, en un sel ou un sel obtenu en un autre sel ou en un composé de formule I à l'état libre, et/ou si on le désire, on isole un isomère optique à partir d'un mélange de formes stéréoisomères d'un composé obtenu répondant à la formule I ou de l'un de ses sels.

2. Procédé selon la revendication 1, caractérisé en ce que l'on fait réagir un composé de formule II

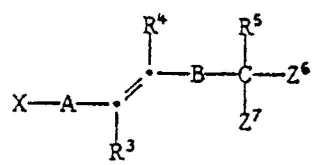

(II)

dans laquelle $R^3$, $R^4$, $R^5$, A et B ont les significations indiquées en référence à la formule I, $Z^6$ représente un groupe carboxy protégé, $Z^7$ représente un groupe amino protégé et X un groupe hydroxy estérifié réactif, avec un composé de formule III

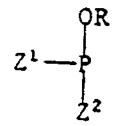

(III)

dans laquelle $Z^1$ représente un groupe hydroxy protégé, $Z^2$ un groupe alkyle inférieur, l'hydrogène protégé ou un groupe hydroxy protégé et R un groupe alkyle inférieur, et on libère ensuite les groupes protégés.

3. Procédé selon la revendication 2, dans lequel $Z^1$ représente un groupe alcoxy inférieur, $Z^2$ un groupe alkyle inférieur, di-(alcoxy inférieur)-alkyle inférieur ou alcoxy inférieur, R un groupe alkyle inférieur, $Z^6$ un groupe (alcoxy inférieur)-carbonyle, $Z^7$ un groupe formylamino et X un halogène.

4. Procédé selon la revendication 1, pour convertir un composé de formule I dans laquelle $R^6$ représente un groupe carboxy en un composé de formule I dans laquelle $R^6$ représente un groupe carboxy estérifié.

5. Procédé selon l'une des revendications 1 à 4, pour la préparation des composés de formule I de la revendication 1 dans laquelle $R^3$ représente l'hydrogène, un groupe alkyle ou aryle.

6. Procédé selon l'une des revendications 1 à 4, pour la préparation des composés de formule I de la revendication 1 dans laquelle $R^6$ représente un groupe (alcoxy inférieur)- ou phényl-(alcoxy inférieur)-carbonyle.

7. Procédé selon l'une des revendications 1 à 4, pour la préparation des composés de formule I dans laquelle $R^1$ représente un groupe hydroxy, alcoxy inférieur ou hydroxyalcoxy inférieur, $R^2$ représente l'hydrogène, un groupe alkyle, hydroxy, alcoxy inférieur ou hydroxyalcoxy inférieur, $R^3$ représente l'hydrogène, un groupe alkyle inférieur, halogénoalkyle inférieur, hydroxyalkyle inférieur, (alcoxy inférieur)-alkyle inférieur, phényl-alkyle inférieur non substitué ou substitué dans la partie phényle, alcényle inférieur, un halogène ou un groupe phényle non substitué ou substitué, $R^4$ représente l'hydrogène, un groupe alkyle inférieur ou un groupe phényle non substitué ou substitué, $R^5$ représente l'hydrogène ou un groupe alkyle inférieur, $R^6$ représente un groupe carboxy ou un groupe carboxy estérifié ou amidé acceptable pour l'usage pharmaceutique, $R^7$ représente un groupe amino, mono- ou di-(alkyle inférieur)-amino, alcanoylamino ou un groupe alcanoylamino substitué par des halogènes, des groupes amino et/ou phényle, carbamoyle, carboxy, imidazolyle, alkylthio inférieurs, tétrahydropyrrolyle, hydroxy, indolyle ou hydroxyphényle, un groupe benzoylamino ou benzoylamino ou phtalimino substitué par des halogènes, des groupes alcoxy inférieurs ou nitro, A représente un groupe alpha,oméga-alkylène en C 1-C 3 non substitué ou substitué par un groupe alkyle inférieur et B représente une liaison, les substituants des groupes phényle étant choisis parmi les groupes alkyle inférieurs, hydroxy, alcoxy inférieurs, les halogènes, les groupes amino, halogénoalkyle inférieurs, hydroxyalkyle inférieurs, aminoalkyle inférieurs et nitro, et de leurs sels acceptables pour l'usage pharmaceutique.

8. Procédé selon la revendication 7, pour la préparation des composés de formule I dans laquelle $R^6$ représente un groupe carboxy, alcoxycarbonyle ou alcoxycarbonyle substitué par des groupes amino, mono- ou di-(alkyle inférieur)-amino, hydroxy ou alcanoyloxy inférieur, $R^7$ représente un groupe amino, mono-(alkyle inférieur)-amino, alcanoylamino inférieur ou benzoylamino, et de leurs sels acceptables pour l'usage pharmaceutique.

9. Procédé selon la revendication 8, pour la préparation des composés de formule I dans laquelle $R^3$ et $R^4$ représentent chacun, indépendamment l'un de l'autre, l'hydrogène, un groupe alkyle inférieur, phényle ou phényle substitué par des groupes alkyle inférieurs, hydroxy, alcoxy inférieurs, des halogènes, des groupes amino, halogénoalkyle inférieurs, hydroxyalkyle inférieurs, aminoalkyle inférieurs ou nitro, et de leurs sels acceptables pour l'usage pharmaceutique.

10. Procédé selon l'une des revendications 1 à 4, pour la préparation des composés de formule I dans laquelle $R^1$ représente un groupe hydroxy ou alcoxy inférieur, $R^2$ représente l'hydrogène, un groupe alkyle, hydroxy ou alcoxy inférieur, $R^3$ représente l'hydrogène, un groupe alkyle inférieur, phényle,

halogénophényle ou phényl-alkyle inférieur, $R^4$ et $R^5$ représentent l'hydrogène ou un groupe alkyle inférieur, $R^6$ représente un groupe carboxy, alcoxycarbonyle ou hydroxy-(alcoxy inférieur)-carbonyle, $R^7$ représente un groupe amino, mono-(alkyle inférieur)-amino, alcanoylamino inférieur ou benzoylamino, A représente un groupe alpha,oméga-alkylène en C 1-C 3 non substitué ou substitué par un groupe alkyle inférieur et B représente une liaison, et de leurs sels acceptables pour l'usage pharmaceutique.

**11.** Procédé selon l'une des revendications 1 à 4, pour la préparation des composés de formule I dans laquelle $R^1$ représente un groupe hydroxy, $R^2$ représente l'hydrogène, un groupe alkyle ou hydroxy, $R^3$ représente l'hydrogène, un groupe alkyle inférieur ou halogénophényle, $R^4$ représente l'hydrogène ou un groupe halogénophényle, et $R^5$ représente l'hydrogène, $R^6$ représente un groupe carboxy, (alcoxy inférieur)-carbonyle ou hydroxy-(alcoxy inférieur)-carbonyle, $R^7$ représente un groupe amino, mono-(alkyle inférieur)-amino, alcanoylamino inférieur ou benzoylamino, A représente un groupe alpha,oméga-alkylène en C 1-C 3 et B une liaison, et de leurs sels acceptables pour l'usage pharmaceutique.

**12.** Procédé selon l'une des revendications 1 à 4, pour la préparation des composés de formule I dans laquelle $R^1$ représente un groupe hydroxy, $R^2$ représente l'hydrogène, un groupe alkyle inférieur ou hydroxy, $R^3$ représente l'hydrogène ou un groupe alkyle inférieur, $R^4$ et $R^5$ représentent l'hydrogène, $R^6$ représente un groupe carboxy ou (alcoxy inférieur)-carbonyle, $R^7$ représente un groupe amino ou mono-(alkyle inférieur)-amino, A représente un groupe alpha,oméga-alkylène en C 1-C 3 et B une liaison, et de leurs sels acceptables pour l'usage pharmaceutique.

**13.** Composés de formule I de la revendication 1 dans laquelle $R^1$ et $R^2$ représentent des groupes hydroxy, $R^3$ l'hydrogène ou un groupe alkyle inférieur, $R^4$ et $R^5$ l'hydrogène, $R^6$ un groupe carboxy, $R^7$ un groupe amino, A un groupe méthylène et B une liaison, et de leurs esters alkyliques inférieurs d'acides carboxyliques et sels acceptables pour l'usage pharmaceutique.

**14.** Procédé selon l'une des revendications 1 à 4, pour la préparation de l'acide E-2-amino-5-phosphono-3-penténoïque et de ses sels acceptables pour l'usage pharmaceutique.

**15.** Procédé selon l'une des revendications 1 à 4, pour la préparation de l'acide E-2-amino-4-méthyl-5-phosphono-3-penténoïque et de ses sels acceptables pour l'usage pharmaceutique.

**16.** Procédé selon l'une des revendications 1 à 4, pour la préparation de l'acide (D)-E-2-amino-4-méthyl-5-phosphono-3-penténoïque et de ses sels acceptables pour l'usage pharmaceutique.

**17.** Procédé selon l'une des revendications 1 à 4, pour la préparation de l'acide E-2-amino-4-éthyl-5-phosphono-3-penténoïque et de ses sels acceptables pour l'usage pharmaceutique.

**18.** Procédé selon l'une des revendications 1 à 4, pour la préparation de l'acide E-2-amino-4-propyl-5-phosphono-3-penténoïque et de ses sels acceptables pour l'usage pharmaceutique.

**19.** Procédé selon l'une des revendications 1 à 4, pour la préparation de l'acide E-2-amino-4-butyl-5-phosphono-3-penténoïque et de ses sels acceptables pour l'usage pharmaceutique.

**20.** Procédé selon l'une des revendications 1 à 4, pour la préparation de l'acide E-2-amino-4-isopropyl-5-phosphono-3-penténoïque et de ses sels acceptables pour l'usage pharmaceutique.

**21.** Procédé selon l'une des revendications 1 à 4, pour la préparation de l'acide E-2-amino-4-benzyl-5-phosphono-3-penténoïque et de ses sels acceptables pour l'usage pharmaceutique.

**22.** Procédé selon l'une des revendications 1 à 4, pour la préparation du E-2-amino-4-méthyl-5-phosphono-3-penténoated'éthyle et de ses sels acceptables pour l'usage pharmaceutique.

**23.** Procédé selon l'une des revendications 1 à 4, pour la préparation du E-2-amino-4-méthyl-5-phosphono-3-penténoatede méthyle et de ses sels acceptables pour l'usage pharmaceutique.

**24.** Procédé selon l'une des revendications 1 à 4, pour la préparation du E-2-amino-4-méthyl-5-phosphono-

3-penténoatede n-propyle et de ses sels acceptables pour l'usage pharmaceutique.

25. Procédé selon l'une des revendications 1 à 4, pour la préparation du E-2-amino-4-méthyl-5-phosphono-3-penténoatede n-butyle et de ses sels acceptables pour l'usage pharmaceutique.

26. Procédé selon l'une des revendications 1 à 4, pour la préparation du E-2-amino-4-méthyl-5-phosphono-3-penténoated'isobutyle et de ses sels acceptables pour l'usage pharmaceutique.

27. Procédé selon l'une des revendications 1 à 4, pour la préparation du E-2-amino-4-méthyl-5-phosphono-3-penténoatede n-pentyle et de ses sels acceptables pour l'usage pharmaceutique.

28. Procédé selon l'une des revendications 1 à 4, pour la préparation du E-2-amino-4-méthyl-5-phosphono-3-penténoatede n-hexyle et de ses sels acceptables pour l'usage pharmaceutique.

29. Procédé de préparation de compositions pharmaceutiques, caractérisé en ce que l'on mélange un composé obtenu selon l'une des revendications 1 à 28 avec un véhicule acceptable pour l'usage pharmaceutique.